# FASCICULE DE BREVET EUROPEEN

(11) **EP 2 651 453 B1**
(45) Date de publication et mention de la délivrance du brevet: **22.11.2017**
(21) Numéro de dépôt: 11788849.5
(22) Date de dépôt: 30.11.2011
(51) Int. Cl.: A61K 49/00, A61K 49/14, A61K 49/18, A61K 51/08, A61K 51/04, A61K 49/04

(54) **COMPOSÉS POUR LE DIAGNOSTIC DE MALADIES LIÉES À L'EXPRESSION DE MUC5AC**
VERBINDUNGEN ZUR DIAGNOSE VON ERKRANKUNGEN IN ZUSAMMENHANG MIT DER EXPRESSION VON MUC5AC
COMPOUNDS FOR DIAGNOSING DISEASES ASSOCIATED WITH THE EXPRESSION OF MUC5AC

(30) Priorité: 14.12.2010 FR 1060497
(43) Date de publication de la demande: 23.10.2013
(73) Titulaire: Guerbet, 93420 Villepinte (FR)
(72) Inventeur: BALLET, Sébastien, F-75011 Paris (FR); GONZALEZ, Walter, F-93270 Sevran (FR); ROSSEZ, Yannick, F-59310 Faumont (FR)
(74) Mandataire: Lavoix
(86) Numéro de dépôt international: PCT/EP2011/071427
(87) Numéro de publication internationale: WO 2012/079989

(56) Documents cités:
- WO-A2-03/086284
- DEUTSCHER SUSAN L: "Phage display in molecular imaging and diagnosis of cancer.", CHEMICAL REVIEWS 12 MAY 2010 LNKD- PUBMED:20170129, vol. 110, no. 5, 12 mai 2010 (2010-05-12), pages 3196-3211, XP002644582, ISSN: 1520-6890
- KELLY KIMBERLY A ET AL: "Isolation of a colon tumor specific binding peptide using phage display selection", NEOPLASIA, NEOPLASIA PRESS, ANN ARBOR, MI, US, vol. 5, no. 5, 1 octobre 2003 (2003-10-01) , pages 437-444, XP002411411, ISSN: 1522-8002
- HSIUNG PEI-LIN ET AL: "Detection of colonic dysplasia in vivo using a targeted heptapeptide and confocal microendoscopy", NATURE MEDICINE, vol. 14, no. 4, avril 2008 (2008-04), pages 454-458, XP002644583, ISSN: 1078-8956
- LIU D ET AL: "Comparative evaluation of three <64>Cu-labeled E. coli heat-stable enterotoxin analogues for PET imaging of colorectal cancer", BIOCONJUGATE CHEMISTRY 20100721 AMERICAN CHEMICAL SOCIETY USA LNKD- DOI:10.1021/BC900513U, vol. 21, no. 7, 21 juillet 2010 (2010-07-21), pages 1171-1176, XP002644584, ISSN: 1043-1802
- LI Z J ET AL: "A novel peptide specifically targeting the vasculature of orthotopic colorectal cancer for imaging detection and drug delivery", JOURNAL OF CONTROLLED RELEASE 20101220 ELSEVIER NLD LNKD- DOI:10.1016/J.JCONREL.2010.09.015, vol. 148, no. 3, 18 septembre 2010 (2010-09-18), pages 292-302, XP002644585, ISSN: 0168-3659
- KELLY KIMBERLY ET AL: "Detection of invasive colon cancer using a novel, targeted, library-derived fluorescent peptide", CANCER RESEARCH, AMERICAN ASSOCIATION FOR CANCER REREARCH, US, vol. 65, no. 17, 1 septembre 2004 (2004-09-01), pages 6247-6251, XP009113912, ISSN: 0008-5472, DOI: DOI:10.1158/0008-5472.CAN-04-0817
- BYRD J C ET AL: "Mucins and mucin binding proteins in colorectal cancer", CANCER AND METASTASIS REVIEWS 200401 NL LNKD- DOI:10.1023/A:1025815113599, vol. 23, no. 1-2, janvier 2004 (2004-01), pages 77-99, XP002644586, ISSN: 0167-7659
- FORGUE-LAFITTE M -E ET AL: "Abnormal expression of M1/MUC5AC mucin in distal colon of patients with diverticulitis, ulcerative colitis and cancer", INTERNATIONAL JOURNAL OF CANCER 20071001 US LNKD- DOI:10.1002/IJC.22865, vol. 121, no. 7, 1 octobre 2007 (2007-10-01), pages 1543-1549, XP002644587, ISSN: 0020-7136
- LEE SEULKI ET AL: "Peptides and peptide hormones for molecular imaging and disease diagnosis.", CHEMICAL REVIEWS 12 MAY 2010 LNKD- PUBMED:20225899, vol. 110, no. 5, 12 mai 2010 (2010-05-12), pages 3087-3111, XP002644588, ISSN: 1520-6890

## Description

L'invention concerne de nouveaux composés de diagnostic de maladies liées à l'expression de MUC5AC, leur procédé de préparation et leur utilisation en imagerie médicale.
Le cancer colorectal est caractérisé par une perte de différenciation des cellules composant les glandes de Lieberkühn. Ces glandes tapissant la paroi du tube digestif sont responsables de la synthèse du mucus dont les composants majeurs sont les mucines. Les mucines constituent une vaste famille d'O-glycoprotéines codées par les gènes *MUC.* Ce sont des composés très hétérogènes, de haute masse moléculaire et très riches en sucres (jusqu'à 85% de la masse totale). Elles sont formées d'un axe peptidique qui peut être hérissé de plusieurs centaines de chaînes glycanniques différentes. Les mucines intestinales exercent un rôle de lubrifiant et protègent l'épithélium face aux agressions mécaniques associées au processus de digestion. Elles établissent, par l'intermédiaire de leurs chaînes glycanniques, de nombreuses interactions avec les microorganismes, permettant ainsi de maintenir une flore bactérienne riche et diversifiée, tout en empêchant la prolifération de souches pathogènes. Il a en effet été proposé que les différents oligosaccharides constituent une mosaïque de récepteurs potentiels capables de piéger de nombreux microorganismes dans la couche de mucus en empêchant ainsi l'infection de l'épithélium sous-jacent.
De nombreuses études ont mis en évidence des anomalies d'expression et des modifications de la glycosylation des mucines chez les patients atteints de cancer du côlon qui influenceraient la croissance cellulaire, la différenciation, la transformation, l'adhésion et l'invasion des cellules tumorales. La glycoprotéine MUC2 est la mucine majoritaire de l'intestin grêle au rectum. Son expression et celle de MUC3 sont fortement diminuées dans certains adénomes colorectaux alors que l'on peut observer une expression *de novo* de MUC5AC.
Dans des conditions physiologiques, MUC5AC est présente dans l'appareil respiratoire, la muqueuse gastrique et génitale et n'est pas présente dans la muqueuse colique saine. En revanche, elle est exprimée de façon aberrante par certaines tumeurs et lésions pré-cancéreuses colorectales. Ainsi, les tumeurs villeuses peu dysplasiques réagissent positivement aux anticorps anti-MUC5AC mais l'expression de cette mucine diminue lorsque le grade de dysplasie augmente et disparaît totalement quand la tumeur se transforme en adénocarcinome. Il a par ailleurs été montré que 60% des adénomes « classiques » (tubuleux et villeux) sont porteurs de MUC5AC contre 100% des adénomes dentelés (*serrated adenoma*) et des polypes hyperplasiques.

L'ensemble de ces données indique que MUC5AC est un marqueur précoce de transformation cancéreuse au niveau de la muqueuse colique.

Aujourd'hui encore, un patient sur deux atteint du cancer du côlon décède en France et c'est le stade d'avancement de la tumeur au moment du diagnostic qui est déterminant pour le pronostic. Peu de méthodes diagnostiques du cancer colorectal existent, puisque seules la coloscopie et la recherche d'hémoglobine dans les selles sont employées. La coloscopie est actuellement la méthode de screening de choix, mais il s'agit d'un examen réputé pénible et donc souvent mal accepté. D'autant qu'elle ne permet pas de détecter les polypes néoplasiques plans ou sessiles qui sont par définition peu visibles et donc souvent ignorés lors d'un examen endoscopique classique. L'examen des selles ne permet, quant à lui, que rarement la découverte d'adénomes, même à un stade avancé, et ne se prête donc pas à la prévention du développement du cancer. C'est pour cela que des modalités d'imagerie connues de l'homme du métier sont des alternatives intéressantes, comme l'IRM, les rayons X, la scintigraphie aux rayons gamma, le scanner CT, les ultrasons, le PET, l'imagerie optique. On rappelle que dans le cas de l'IRM, un contraste est obtenu grâce à l'administration d'agents de contraste contenant des métaux paramagnétiques ou superparamagnétiques qui ont un effet sur la relaxivité des protons de l'eau. Dans le cas de la scintigraphie, le contraste est obtenu par la localisation spécifique d'un composé radiopharmaceutique émettant des rayons 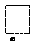 ou □.
On recherche des composés dont la synthèse chimique n'est pas trop complexe, suffisamment stables *in vivo* pour une utilisation en imagerie médicale, et dont le prix de revient n'est pas trop élevé, et cela notamment pour l'IRM.

Le demandeur a réussi à obtenir des composés ciblant spécifiquement la mucine MUC5AC, et très avantageux pour l'imagerie diagnostique. En particulier le demandeur a obtenu des résultats favorables notamment *in vitro* sur la mucine purifiée, sur des cellules en culture et sur des coupes histologiques. Des peptides ont été sélectionnés pour reconnaitre MUC5AC quelque soit sa glycosylation et sans marquer d'autres mucines comme MUC2 qui sont des glycoprotéines très proches structurellement.
Les composés efficaces ont à la fois une affinité suffisante pour reconnaître la cible, une forte spécificité pour être un indicateur distinctif de l'état pathologique, une stabilité appropriée pour ne pas être dégradés ou modifiés *in vivo* ; et en plus sans que la partie de signal attachée au peptide n'interfère de manière gênante sur ces différents paramètres (affinité et stabilité en particulier).

Après de nombreuses tentatives, le demandeur a réussi à obtenir des composés efficaces. L'invention concerne ainsi un composé de formule générale (I) suivante :
Signal-Lien-Peptide (I)
   Dans laquelle :
   - Signal représente une entité signal ;
   - Lien, présent ou non, représente une partie de liaison chimique et
   - Peptide représente un peptide comprenant un peptide ciblant MUC5AC (plus exactement une partie peptidique comprenant ou constituée par au moins un peptide ciblant MUC5AC), le peptide ciblant MUC5AC étant choisi parmi les peptides de formule suivante, et leurs équivalents fonctionnels :
      a) X1-X2-X3-X4-X5-X6-X7-X8-X9 (1) (SEQ ID No1) avec :
         X1 est absent ou choisi parmi la cystéine, la méthionine
         X2 choisi parmi l'hydroxyproline ou la proline
         X3 choisi parmi la thréonine ou la sérine
         X4 choisi parmi l'isoleucine, la leucine, la valine
         X5 choisi parmi la tyrosine, la phénylalanine, le tryptophane
         X6 choisi parmi l'hydroxyproline ou la proline
         X7 choisi parmi l'isoleucine, la leucine, la valine
         X8 choisi parmi l'isoleucine, la leucine, la valine
         X9 est absent ou choisi parmi la cystéine, la méthionine , de préférence le peptide CPSIYPLLC (SEQ ID No2) (Cys-Pro-Ser-Ile-Tyr-Pro-Leu-Leu-Cys) et le peptide PSIYPLL (SEQ ID No3) ;
      b) X10-X11-X12-X13-X14-X15-X16-X17-X18 (2) (SEQ ID No4) avec:
         X10 est absent ou choisi parmi la cystéine, la méthionine
         X11 choisi parmi l'isoleucine, la leucine, la valine, alanine
         X12 choisi parmi la thréonine ou la sérine
         X13 choisi parmi l'isoleucine, la leucine, la valine
         X14 choisi parmi l'hydroxyproline ou la proline
         X15 choisi parmi l'hydroxyproline ou la proline
         X16 choisi parmi l'isoleucine, la leucine, la valine
         X17 choisi parmi l'isoleucine, la leucine, la valine
         X18 est absent ou choisi parmi la cystéine, la méthionine
      c) CALIPPLLC (SEQ ID No5) ;
de préférence le peptide CALIPPLLC (SEQ ID No5) (Cys-Ala-Leu-Ile-Pro-Pro-Leu-Leu-Cys) ; les sels pharmaceutiquement acceptables de ces composés du a) ou du b).

L'expression « peptide ciblant MUC5AC » est également désignée PEPTIDE MUC5AC dans la demande. Avantageusement Peptide représente un PEPTIDE MUC5AC.
De façon avantageuse, le Peptide (peptide comprenant un PEPTIDE MUC5AC) selon l'invention comporte au plus 20 aminoacides, avantageusement au plus 15 aminoacides, avantageusement au plus 10 aminoacides, avantageusement 5 à 10 aminoacides.
Par l'expression peptide CPSIYPLLC (SEQ ID No2), PSIYPLL (SEQ ID No3) et leurs équivalents fonctionnels, on entend le peptide CPSIYPLLC (SEQ ID No2), PSIYPLL (SEQ ID No3) dont le demandeur a démontré l'efficacité, et les peptides de formules X1-X2-X3-X4-X5-X6-X7-X8-X9 (1) (SEQ ID No1) dérivés qui une fois inclus dans le composé (I) présentent une efficacité en imagerie semblable ou meilleure que le CPSIYPLLC (SEQ ID No2) (ce qui inclut les peptido-mimétiques), cette efficacité étant testée à l'aide de tests et de modèles in vitro décrits en détail dans la demande ou de modèles analogues appropriés. Le peptide CPSIYPLLC (SEQ ID No2) étudié est un peptide cyclique et est exemplifié en détail dans la demande.
De même par l'expression CALIPPLLC (SEQ ID No5) et ses équivalents fonctionnels, on entend le peptide CALIPPLLC (SEQ ID No5) dont le demandeur a démontré l'efficacité, et les peptides de formule X10-X11-X12-X13-X14-X15-X16-X17-X18 (2) (SEQ ID No4) dérivés efficaces. Le peptide CALIPPLLC (SEQ ID No5) étudié est un peptide cyclique et est exemplifié en détail dans la demande.
En particulier, un dérivé du peptide CPSIYPLLC (SEQ ID No2) et PSIYPLL (SEQ ID No3) inclut un peptide ou composé dans lequel la séquence PSIYPLL (SEQ ID No3) constituant la séquence a été modifiée par ajout, suppression, substitution ou modification d'au moins un acide aminé.
La substitution peut être conservative ou non. La substitution est conservative lorsqu'un acide aminé est substitué par un acide aminé ayant des propriétés similaires (par exemple, polarité, potentiel de liaison hydrogène, acidité, basicité, hydrophobicité, présence d'un groupe aromatique, etc). Un acide aminé naturel peut être remplacé par un acide aminé non naturel, tel qu'un acide aminé en configuration D, acide aminé bêta ou gamma. Le PEPTIDE MUC5AC est par exemple modifié en utilisant des méthodologies appropriées décrites dans l'art antérieur par exemple dans US2005100963 (colonne 20-21 paragraphes [529] à [541] dans le cas de peptides ciblant des récepteurs KDR), afin de sélectionner des composés (I) efficaces.

Dans le cas de CPSIYPLLC (SEQ ID No2) et PSIYPLL (SEQ ID No3)
- P la proline peut être remplacée par un autre acide aminé porteur d'un hétérocycle par exemple l'hydroxyproline, l'histidine
- S la sérine peut être remplacée par un autre acide aminé porteur d'un groupe hydroxy, par exemple la thréonine, l'homosérine
- I l'isoleucine peut être remplacée par d'autres acides aminés aliphatiques (leucine, valine) ou des dérivés, par exemple substitués par des alkyles.
- Y tyrosine peut être remplacée par d'autres acides aminés aromatiques notamment le tryptophane, la phénylalanine
- L la leucine peut être remplacée par d'autres acides aminés aliphatiques (isoleucine, valine)

La séquence PSIYPLL (SEQ ID No3) peut être modifiée en remplaçant une ou plusieurs liaisons amide par une liaison conférant une stabilité accrue in vivo, par exemple conférant une résistance accrue à la protéolyse.
On entend par peptide comprenant au moins un peptide ciblant MUC5AC, un peptide présentant cette séquence peptidique de reconnaissance de la cible biologique, éventuellement flanquée en extrémité N et/ou C terminal d'un groupe chimique n'interférant pas sur l'efficacité en imagerie de cette séquence.

On entend par le terme Signal ou entité signal une entité chimique permettant d'obtenir en imagerie médicale, en particulier :
- un chélate capable d'être couplé à un métal paramagnétique
- une nanoparticule métallique, notamment une nanoparticule superparamagnétique d'oxyde de fer
- une nanoparticule lipidique, avantageusement sous forme d'émulsion, cette nanoparticule étant porteuse d'au moins un chélate capable d'être couplé à un métal paramagnétique (dans le cas, le peptide est greffé à la nanoparticule lipidique en émulsion qui est elle-même porteuse de chélates ; la liaison du peptide à la nanoparticule lipidique se fait par exemple par un groupe de liaison chimique)
On entend dans la formule I par l'expression « partie de liaison chimique », un ou plusieurs groupes de liaison ou liens L, c'est-à-dire un groupe chimique :
- permettant de relier le Signal et la partie peptidique PEPTIDES MUC5AC
- n'ayant pas lui-même la fonction d'entité signal qui est assurée par le signal
- n'ayant pas lui-même la fonction de ciblage qui est assurée par le PEPTIDE MUC5AC.

Selon un mode de réalisation, l'entité signal comprend un chélate (sous forme complexée ou sous forme complexée avec un métal M). Avantageusement le chélate est couplé au métal M. Un grand nombre de chélates, de préférence macrocycliques, sont utilisables, notamment de formule suivante (illustration avec le gadolinium Gd, d'autres lanthanides étant aussi appropriés) 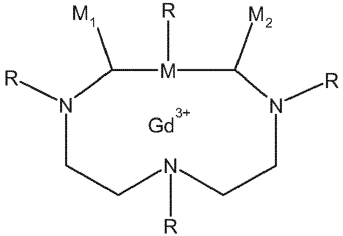 avec :
M-M1-M2 forme un noyau pyridine
ou M1 et M2 sont absents et M représente une liaison
ou M est N-R et M1 et M2 représentent un atome d'hydrogène ou un méthyle avec R est choisi indépendamment parmi CH₂CO₂- ou H ou CHX-CO₂- , avec au moins un R étant CHXCO₂- et X étant L-B ;
On pourra utiliser notamment un chélate macrocyclique parmi 1,4,7,10-tetraazacyclododecan-1,4,7,10-tetraacetic acide (DOTA), 1,4,7,10-tetraazacyclododecan-1,4,7-triacetic acide (DO3A), 10-(2-hydroxypropyl)-1,4,7,10-tetraazacyclododecan-1,4,7-triacetic acide (HPDO3A), (MCTA), (DOTMA), 3,6,9,15-tetraazabicyclo[9.3.1]pentadeca-1(15),11,13-triene-3,6,9-triacetic acide (PCTA).
On pourra aussi utiliser des dérivés dans lesquels un ou plusieurs groupes carboxyliques sont sous forme d'un sel, ester, amide correspondant ; ou un composé correspondant dans lequel un ou plusieurs groupes carboxyliques sont remplacés par un groupe phosphonique et/ou phosphinique.
On peut aussi utiliser un chélate parmi : DOTA gadofluorines, DO3A, HPDO3A, TETA, TRITA, HETA, DOTA-NHS, M4DOTA, M4DO3A, PCTA, BOPTA et leurs dérivés.
De manière plus large, le ou les chélates formant l'entité signal pourront répondre à la formule du document WO01/60416 ou WO03/062198 (page 23 à 25). 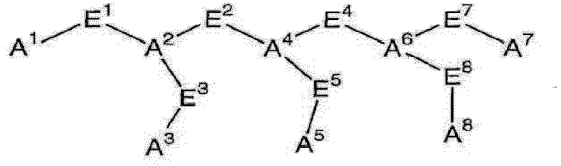 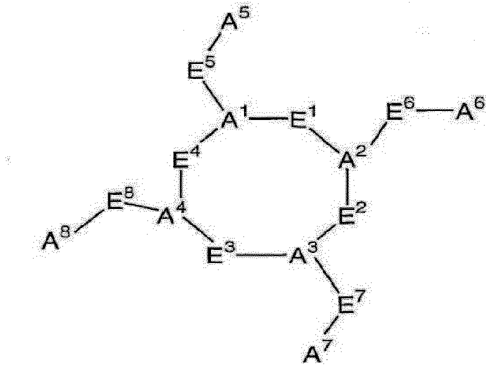 On pourra utiliser en particulier les composés DOTA, NOTA, DO3A, AAZTA, HOPO, ainsi que leur multimères et dérivés connus notamment : 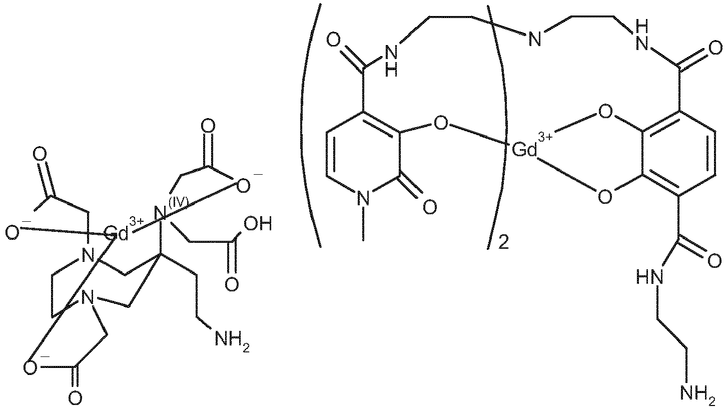 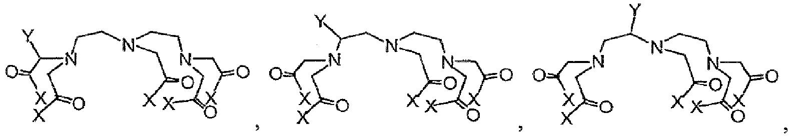 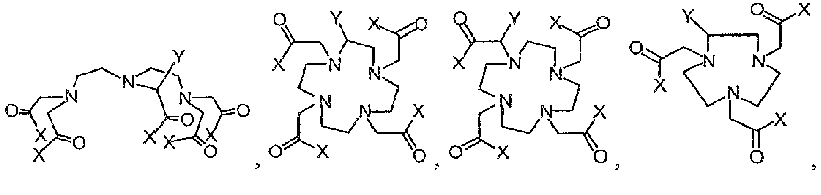 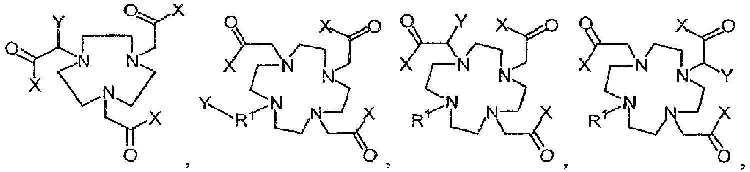 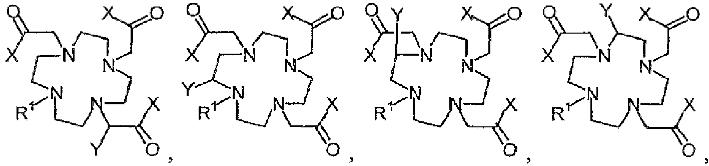 and 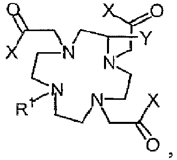 Le couplage de chélates avec des biovecteurs en particulier peptidiques est décrit dans l'art antérieur, et fait généralement intervenir une partie de liaison chimique (Lien) telle que décrite par exemple dans le document WO01/60416.

La structure et la nature chimique de la partie de liaison chimique sont définies pour permettre le couplage chimique entre d'une part la partie peptidique PEPTIDE MUC5AC et d'autre part l'entité Signal (le ou les chélates utilisés), et de manière à obtenir une affinité de la partie PEPTIDE MUC5AC pour sa cible et une spécificité de la reconnaissance appropriée pour l'utilisation.

Avantageusement, le métal M est un ion de métal paramagnétique, ou un radionucléide. Le complexe formé par le chélate et le métal M est stable dans les conditions physiologiques de manière à éviter une libération non souhaitée du métal M dans l'organisme. Avantageusement l'entité signal (chélate) comprend au moins un groupe fonctionnel permettant de relier l'entité signal avec le lien (la partie de liaison chimique décrite dans la demande) ou directement avec le Peptide. L'invention concerne aussi les composés de formule (I) dans lesquels le chélate n'est pas complexé avec le métal.

Un grand nombre de Lien entre le ou les chélates et les ou les PEPTIDES MUC5AC peuvent être utilisés, dans la mesure où ils sont capables d'interagir avec au moins un groupe fonctionnel de peptide et au moins un groupe fonctionnel de chélate. Avantageusement le groupe de liaison chimique ou lien L est choisi parmi les suivants :
a) Un groupe de formule Q1-I-Q2, dans laquelle Q1 et Q2 identiques ou différents représentent O, S, NH, CO2, -NHCO, CONH, NHCONH, NHCSNH, SO2NH- ou NHSO2-, et I représente un groupe alkyle (avantageusement en C₁-C₁₀), alcoxyalkyle (avantageusement en C₁-C₁₀), alcényle (avantageusement en C₂-C₆), alcynyle (avantageusement en C₂-C₆), polyalkoxyalkylène, alkyle interrompu par un ou plusieurs squarate, par un ou plusieurs aryles, avantageusment phényle, ou par un ou plusieurs groupes choisis parmi -NH-, -O-, -CO-, -NH(CO)-, -(CO)NH-, - O(CO)-, ou -(OC)O- ;
b) Un groupe (CH₂)ₙ, (CH₂)ₙ-CO-, -(CH₂)ₙNH-CO- avec n = 1 à 10, (CH₂CH₂O)_{q}(CH₂)ᵣ-CO-, (CH₂CH₂O)_{q}(CH₂)ᵣ-NH-CO- avec q = 1-10 et r = 1-10, (CH2)n-CONH-, (CH₂)ₙCONH-PEG, (CH2)ₙ-, squarate, 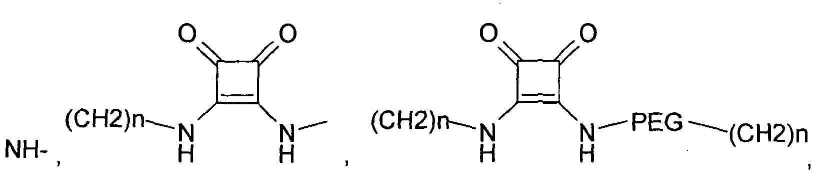 (CH2)n-squarate- (CH₂CH₂O)_{q}(CH₂)ᵣ-CO avec n = 1 à 5 et avantageusement n = 3, 4, 5, HOOC-CH₂-O-(CH₂)₂-O-CH₂-COOH ; HOOC-(CH₂)₂-CO₂-(CH₂)₂-OCO-(CH₂)₂-COOH ; HOOC-CH(OH)-CH(OH)-COOH ; HOOC-(CH₂)ₙ-COOH ; NH₂(CH₂)n-NH₂, avec n = 1-20 ; NH₂-(CH₂)ₙCO₂H ; NH₂-CH₂(CH₂-O-CH₂)ₙ-CO₂H avec n = 1 à 10.
   Notamment : 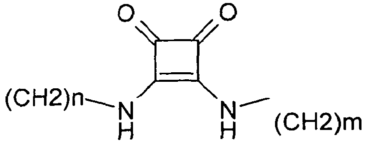 avec n=1 à 4 et m = 0 à 5 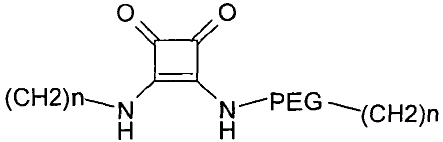 avec n=1 à 4 et m = 0 à 5 (CH2)n - CO avec n = 1 à 5
   (CH₂)₃ - squarate - (CH₂CH₂O)₂(CH₂)-CO
c) Des liens décrits dans les brevets US 6 264 914, capables de réagir avec les groupes fonctionnels (du biovecteurs et du chélate) amino, hydroxyle, sulfhydryle, carboxyle, carbonyle, carbohydrates, thioesters, 2-aminoalcohols, 2-aminothiols, guanidyle, imidazolyle, phénolique ; selon les définitions de ce document.
d) Certains liens décrits dans le brevet US 6 537 520 de formule
   - (Cr₆r₇)_{g}-(W)ₕ-(Cr₆ₐr₇ₐ)_{g'}-(Z)ₖ-(W)_{h'}-(Cr₈r₉)_{g"}-(W)_{h"}-(Cr₈ₐr₉ₐ)_{g'''} avec : g+h+g'+k+h'+g"+h"+g''' différent de 0 ; avec les définitions identiques à celles de ce document colonne 8
e) Certains liens décrits dans le document WO 02/085908 (avec les définitions identiques à celles de ce document), par exemple une chaîne linéaire ou ramifiée de liaison organique choisie parmi :
   - CR6'''R7'''-, (R6''')C=C(R7''')=, -CC-, -(O)-, -O-, -S-, -SO0-, -N(R3"')-, - (R6''')C=N-, -C(S)-, -P(O0(OR3''')-, -P(O)-(OR3444)O-, avec R'''3 un groupe capable de réagir avec un azote ou un oxygène
   - une région cyclique (cycloalkyles divalents, hétérocyles divalents)
   - des polyalkylènes, polyalkylènes glycols
f) Des liens du document WO03/011115 pages 124-125
g) Des liens du document US 2006/0018830

Le choix de Lien (structure et taille) pourra se faire notamment de manière à contrôler la charge, la lipophilie, l'hydrophilie du produit de formule (I), pour optimiser le ciblage biologique, la biodistribution. On pourra en particulier utiliser des liens biodégradables *in vivo*, des liens PEG ou mini-PEG. Le choix du lien est effectué de manière à ne pas altérer de manière gênante l'efficacité du composé de formule (I) selon l'invention, un test permettant de vérifier cette efficacité in vitro étant présenté dans la description détaillée.

Selon un autre mode de réalisation, signal représente un marqueur pour imagerie optique (molécule fluorescente utilisée en imagerie optique, notamment un composé connu pour avoir un maximum d'absorption ou d'émission entre 450 et 1500 nm). Des colorants représentant l'entité Signal et adaptés pour l'imagerie optique sont connus de l'homme du métier, et par exemple choisis parmi les suivants ou leurs dérivés : cyanines, mérocyanines, indocyanines, phthalocyanines, naphthalocyanines, triphénylméthines, porphyrines, colorants pyrilium, thiapyrilium, squarylium, croconium, azulenium, indoanilines, benzophenoxazinium, benzothiaphenothiazinium, anthraquinones, napthoquinones, indathrènes, phthaloylacridones, traphénoquinones, azo, tropones, tétrazines, fé(dithiolène), fos(benzène- dithiolate), iodoaniline, 6/s(S,O-dithiolène), des chomophores notamment fluorescéine, sulforhodamine 101 (Texas Red), rhodamine B, rhodamine 6G, rhodamine 19, indocyanine, Cy2, Cy3B, Cy3.5, Cy5, Cy5.5, Cy7, Cy7.5. On cite notamment les structures suivantes :

| **Type** | **Structure** |
|---|---|
| fluorescéine | 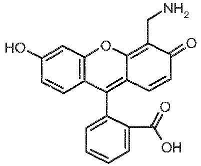 |
| Rhodamine | 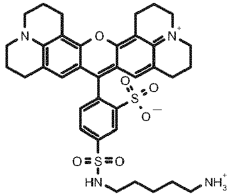 |
| Cyanine | 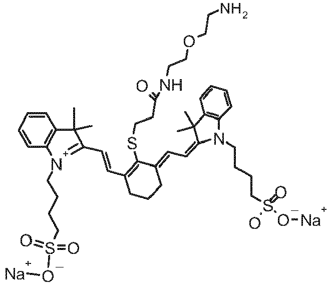 |
| Cyanine | 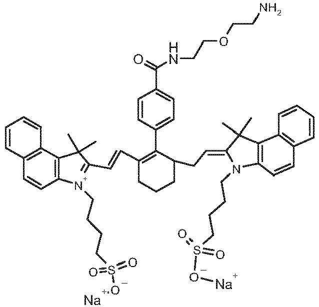 |

Selon un autre mode de réalisation, Signal représente des quantum dots (fluorophores inorganiques comprenant des nanocristaux).
Selon un autre mode de réalisation, Signal représente une nanoparticule superparamagnétique revêtue d'une couche organique, communément désignée SPIO ou USPIO (« ultra small particles of iron oxide »). Avantageusement, la nanoparticule comporte un noyau d'oxyde ou d'hydroxyde de fer, notamment de magnétite (Fe₃O₄), maghémite (□-Fe₂O₃). On utilisera avantageusement une nanoparticule recouverte d'un revêtement citrate, silane, siloxane, phosphate, phosphonate, bisphosphonate, avantageusement gem-bisphosphonate, décrite dans WO200458275 (et d'autres variantes de tels revêtements par exemple décrits dans WO2007095871, WO2007064175, WO2007099289, US20090208420, WO2008115854), la particule et la méthode de couplage entre le peptide et la nanoparticule étant détaillées dans les exemples de la présente demande. Les nanoparticules magnétiques utilisées sont très avantageusement des nanoparticules à base d'un composé du fer, et recouvertes par une couche comprenant un ou plusieurs composés *gem*-bisphosphonates identiques ou différents, la couche de recouvrement de la nanoparticule ayant la formule (C) suivante :

T-L2-CH(PO₃H₂)₂ (C)

dans laquelle :
- Le lien L2 représente un groupement organique reliant la fonction T à la fonction *gem-*bisphosphonate -CH(PO₃H₂)₂ ;
- T représente une fonction chimique couplée au PEPTIDE MUC5AC ou au lien de la présente demande. Dans un mode de réalisation particulier T-L2 représente le Lien du composé de formule (I).
La composition se présente sous forme d'une solution aqueuse stable de nanoparticules.
Dans ces compositions, le taux de complexation du composé (C) sur les particules est supérieur à 50%, avantageusement à 70%, et de préférence supérieur à 80, 90, 95% (la partie phosphonate se fixe sur le noyau métallique). On préfère particulièrement que les particules magnétiques acides (p) soient complexées sur au moins 90% de leurs sites protonés par des composés de formule (C), une partie de ces composés C étant couplée à des PEPTIDE MUC5AC. Selon une variante, une partie des fonctions T de la couche est couplée à un PEPTIDE MUC5AC, et une partie des fonctions T est couplée à un composé hydrophile notamment un composé porteur de groupes hydroxyle et notamment un composé hydrophile aminoalcool désigné AAG1AA28 décrit dans WO2004058275 (exemple 8) ou un groupe PEG.
Les particules magnétiques (p) possèdent un diamètre hydrodynamique compris entre 5 et 300 nm, de préférence entre 5 et 60 nm, encore de préférence entre 5 et 30 nm Le lien L2 permet de relier et/ou d'espacer la fonction *gem*-bisphosphonate et l'entité réactive T capable d'assurer le greffage covalent du PEPTIDE MUC5AC (le biovecteur) sur la nanoparticule, par l'intermédiaire ou non du Lien.
A titre préféré, le lien L2 représente un groupement divalent.
De préférence, le lien L2 est choisi parmi :
- un groupe aliphatique ; alicyclique ; alicyclique aliphatique ; aromatique ; aromatique aliphatique, lesdits groupes aliphatiques, alicycliques et aromatiques pouvant être éventuellement substitués par un groupe méthyle, hydroxy, méthoxy, acétixy, amido, ou un atome d'halogène, avantageusement un atome de chlore, d'iode ou de brome ;
- un groupement -I₁-NHCO-I₂ où I₁ et I₂, identique ou différents, représentent un groupe aliphatique ; alicyclique ; aromatique ; alicyclique aliphatique ou aromatique aliphatique, lesdits groupes pouvant être éventuellement substitués par un groupe méthyle, hydroxy, méthoxy, acétoxy, amido ou un atome de chlore, d'iode ou de brome.
   Selon des réalisations préférées, L2 représente un groupe aliphatique substitué ou non, et plus préférentiellement un groupe -(CH₂)ₚ-, où p est un entier de 1 à 5, ou préférentiellement un groupe -(CH₂)ₙ-NHCO-(CH₂)ₘ-où n et m représentent un nombre entier de 0 à 5.

Au vu de la demande l'homme du métier comprend que le nombre de groupes chimiques de liaison formant la partie de liaison est variable selon le type de composés I. Par exemple :
- lorsque le Signal est un chélate destiné à être couplé à un seul Peptide, la partie de liaison est avantageusement un groupe chimique reliant le chélate et le Peptide
- lorsque le Signal est un multimère de chélates destiné à être couplé à un Peptide, la partie de liaison est avantageusement un groupe chimique reliant le multimère au Peptide comme cela est connu de l'homme du métier
- lorsque le Signal est une nanoparticule notamment une nanoparticule métallique telle qu'une nanoparticule d'oxyde métallique recouverte de nombreuses (quelques centaines à quelques milliers typiquement) molécules PEPTIDES MUC5AC, chaque PEPTIDE MUC5AC est greffé au noyau d'oxyde métallique par un groupe chimique de liaison comme cela est connu de l'homme du métier
- lorsque le Signal est une nanoparticule lipidique en émulsion, sous forme d'une nanogouttelette lipidique en émulsion formée par un coeur aqueux entourée d'une couche lipidique de tensioactifs sur laquelle sont ancrés des PEPTIDE MUC5AC (plusieurs centaines à plusieurs milliers), chacun de ces PEPTIDES MUC5AC est greffé à cette couche lipidique par un lien chimique.

Les tests biologiques décrits en détail dans la demande permettant de sélectionner des peptides efficaces équivalents ou améliorés en termes d'activité de ciblage de MUC5AC en comparaison avec les peptides exemplifiés en détail dans la présente demande.

L'invention concerne également une méthode de diagnostic utilisant un composé de formule (I) tel que décrit précédemment.
La présente invention concerne également une composition comprenant au moins un composé de formule générale (I) tel que décrit précédemment, avantageusement destiné à une administration par voie parentérale.
L'invention concerne également l'utilisation d'un composé selon la présente invention pour le diagnostic ou la préparation d'une composition de diagnostique d'une pathologie associée à MUC5AC, notamment choisie parmi les maladies héréditaires colorectales (notamment la polypose adénomateuse familiale (PAF) et le syndrome de Lynch), les maladies inflammatoires chroniques du tube digestif notamment chez des sujets atteints depuis de nombreuses années (la recto-colite hémorragique et la maladie de Crohn), les populations risquant de contracter un cancer colorectal, les suivis de récidives chez les patients ayant déjà été traités du cancer colorectal, de façon avantageuse parmi les individus ayant un risque élevé de développer un cancer colorectal et le suivi de récidives de cancer colorectal.
Avantageusement, les états physiologiques favorisant un risque de cancer colorectal sont choisis parmi les maladies héréditaires colorectales associées à la formation de polypes adénomateux colorectaux, les maladies chroniques de l'intestin (MICI), les sujets de plus de 50 ans des 2 sexes et avec un antécédent familial au 1 er degré de cancer ou d'adénome colorectal.
Avantageusement, le suivi de récidive de cancer colorectal est choisi parmi : les individus avec un antécédent personnel d'adénomes développés ou de cancer avant la première intervention.
Pour un diagnostic en IRM, l'administration intraveineuse par injection habituellement en solution saline, se fait typiquement à une dose d'ion métallique de 0,001 à 1,5 mmol/kg de poids corporel, par exemple de 1 à 500µmol Gd/kg. Pour un diagnostique radiopharmaceutique, l'administration intraveineuse par injection habituellement en solution saline, se fait typiquement à une dose de 1 à 100mCi pour 70 kg de poids corporel, de préférence de 5 à 50mCi, avec une imagerie de diagnostic par exemple 30 à 180 minutes après l'injection pour le ⁹⁹Tc.
On rappelle que pour l'IRM, le métal est typiquement un métal paramagnétique, les métaux paramagnétiques incluant les lanthanides de nombre atomique 58-70 et les métaux de transition de nombre atomique 21-29, 42 ou 44, par exemple scandium, titane, vanadium, chrome. Avantageusement le métal paramagnétique est choisi parmi les éléments : manganèse, fer, cobalt, nickel, cuivre, molybdène, ruthénium, cérium, praseodymium, neodymium, prométhium, samarium, europium, gadolinium, terbium, dysprosium, holmium, erbium, thulium, et ytterbium. On préfère particulièrement les éléments Gd(III), Mn(II), europium, dysprosium, avantageusement Gd. Dans le cas d'une utilisation en imagerie multimodale (par exemple IRM + PET) ou en médecine nucléaire (imagerie SPECT et/ou PET), les chélates peuvent être utilisés pour complexer un radioélément tel que notamment ⁹⁹T_{c}, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho, de préférence le technecium, l'Indium, le Gallium.
Pour une utilisation comme agents de contraste aux rayons X, la concentration en atome lourd est typiquement de 0,1 M à 5 M, avec des concentrations par administration intraveineuse de l'ordre de 0,5 à 1,5 mmol/kg.
L'invention concerne selon un autre aspect l'utilisation d'un composé (I) tel que décrit précédemment pour la préparation d'une composition destinée à l'imagerie optique.
Des exemples d'administrations de composition pour l'imagerie médicale sont décrits dans l'art antérieur, par exemple dans le document WO 0226776 et US 2006/0018830 colonne 36 ([0282]) paragraphe 8 (dosages et additives).
Des véhicules pharmaceutiquement physiologiquement acceptables permettant de former des compositions diagnostiques (produit de contraste) comprenant les composés décrits précédemment sont connus de l'art antérieur. On utilisera par exemple des sels (sodium, calcium, méglumine), des agents contrôle de pH (acide acétique, citrique, fumarique, des antioxydants).
L'invention concerne aussi un procédé de préparation de composés comprenant le couplage d'un PEPTIDE MUC5AC avec au moins un chélate. Plusieurs méthodes générales de préparation de composés de formule (I) décrites dans US2006/0018830 (Bracco) sont applicables en utilisant un Peptide à la place des peptides de ces documents. Ces méthodes choisies en fonction notamment du chélate choisi sont rappelées dans 2006/0018830 colonne 37 ([0288] à [0291]) (« general preparation of compounds », et « alternative preparation of the compounds via segment coupling), par exemple les méthodes SPPS et FMOC (carbamate 9-fluorenylmethyl). L'invention concerne aussi une méthode de diagnostic comprenant l'administration d'un composé (I), la réalisation d'un examen d'imagerie à l'aide d'un équipement approprié, et l'analyse des résultats.
L'invention couvre sauf indication contraire, toutes les formes chirales, diastéréoisomères, racémiques, notamment cis-trans, L-D des composés décrits.

Le demandeur a étudié les possibilités d'association d'un PEPTIDE MUC5AC couplé à plusieurs chélates dans le composé de formule (I). Le demandeur a par ailleurs étudié des composés de formule (I) présentant un assemblage entre un ou plusieurs Peptides du composé de formule (I) ciblant MUC5AC, et un ou plusieurs chélates ; et de manière à ce que l'accès à la cible ne soit pas gêné malgré la présence du ou des chélates. Par exemple, le chélate est espacé du ou des PEPTIDES P par le Lien d'une taille suffisante et d'une structure chimique telles que la reconnaissance du ou des peptides par leur cible n'est pas altérée. Parmi les associations biovecteurs (peptides ou éventuels d'autres biovecteurs) /chélates, on peut citer notamment :
- un peptide biovecteur central relié à plusieurs chélates identiques ou différents
- un chélate central relié à plusieurs peptides identiques ou différents
- un premier ensemble [peptides porteur de chélate(s)] couplé par l'intermédiaire d'un lien hydrophobe ou hydrophile à un second ensemble [peptide porteur de chélates], s'écrivant par exemple :
- Ch1-peptide1-Lien-peptide2-Ch2 avec Ch représentant des chélates identiques ou différents et peptide1 et peptide2 représentant des Peptides identiques ou différents
- un ensemble peptide1-(Lien porteur Ch)-peptide2-Ch
On utilisera par exemple la méthode de contraction de composés multimériques décrite dans US2005/0100963 (WO2006/031885 page 66 ligne 25 à page 69 ligne 30) dans le cas de peptides ciblant des récepteur KDR (par exemple à l'aide de la méthode 13 des exemples : « preparation of homodimers and heterodimers »), mais en utilisant le PEPTIDE MUC5AC (on remplacera par exemple les peptides des composés des figures 44 à 47 du US20050100963 par des PEPTIDES MUC5AC). Le composé peut ainsi avantageusement comprendre un peptide couplé à plusieurs chélates, ou un chélate couplé à plusieurs peptides identiques ou différents.
On pourra le cas échéant coupler la partie peptide ou la partie chélate à des groupements chimiques permettant de favoriser la biodistribution, la durée de vie du produit dans le sang.

La spécificité du produit fait référence à sa capacité de se lier à la cible mucine MUC5AC, la spécificité de la liaison exprimée typiquement par des constantes Kd et Ka, la valeur Kd pour les marqueurs cibles étant de préférence voisine de ou inférieure à 100 µM, de préférence inférieure à 10µM, encore de préférence inférieure à 1µM, avantageusement de 1 à 100 nM.

### Définitions :

On reprend ici les définitions de pathologies dont le diagnostic est l'objet de la présente invention.

Par « peptide ciblant MUC5AC » ou « PEPTIDE MUC5AC » on désigne une molécule capable de se lier de manière sélective à MUC5AC.

Par « tumeurs et lésions pré-cancéreuses colorectales » on inclut notamment les états marquant le développement d'un polype colorectal et les complications découlant d'un polype néoplasique et/ou de son évolution vers la formation d'une tumeur colorectale.

Les « maladies héréditaires colorectales » sont des pathologies augmentant la susceptibilité de développer un cancer du côlon et qui sont induites par des mutations portées par l'ADN génomique de l'individu. Les maladies héréditaires colorectales regroupent essentiellement les polyposes adénomateuses familiales et le syndrome de Lynch (HNPCC : Cancer colorectal héréditaire sans polypose).

Un « polype » est une formation en relief faisant saillie sur la muqueuse intestinale qui le plus souvent n'est pas responsable de symptômes digestifs, différents types de polypes existent : les polypes néoplasiques (pouvant conduire au cancer) et les polypes non néoplasiques (ne conduisant pas au cancer). Parmi les polypes néoplasiques, les polypes adénomateux représentent 70 à 75% des polypes colorectaux et sont classés en 3 types selon leurs aspects microscopiques : adénome tubuleux, adénome tubulo-villeux et adénome villeux.

« Polype adénomateux ou adénome » désigne un foyer circonscrit de dysplasie épithéliale correspondant à des anomalies des cellules de la muqueuse colique. La fréquence des adénomes augmente avec l'âge. L'aspect macroscopique d'un adénome peut-être sessile, pédiculé ou plan.

La « dysplasie » est une malformation ou déformation résultant d'une anomalie du développement d'un tissu ou d'un organe.

Les « maladies inflammatoires chroniques du tube digestif » désignent un ensemble de lésions inflammatoires chroniques, de cause inconnue, pouvant atteindre l'intestin grêle, le colon et le rectum. Ces affections ont des manifestations cliniques variées et souvent une évolution chronique peu prévisible. Ceci regroupe la rectocolite hémorragique et la maladie de Crohn. Les lésions inflammatoires sont secondaires à une activation du système immunitaire intestinal en amont de laquelle les facteurs environnementaux (mode de vie, germes intestinaux, virus) et génétiques (prédisposition) ne sont pas encore bien identifiés.

Dans la présente demande on utilise le tableau de correspondance suivant.

| | | |
|---|---|---|
| Alanine | A | Ala |
| Arginine | R | Arg |
| Asparagine | N | Asn |
| Aspartate | D | Asp |
| Cystéine | C | Cys |
| Glutamate | E | Glu |
| Glutamine | Q | Gln |
| Glycine | G | Gly |
| Histidine | H | His |
| Isoleucine | I | Ile |
| Leucine | L | Leu |
| Lysine | K | Lys |
| Méthionine | M | Met |
| Phénylalanine | F | Phe |
| Proline | P | Pro |
| Sérine | S | Ser |
| Thréonine | T | Thr |
| Tryptophane | W | Trp |
| Tyrosine | Y | Tyr |
| Valine | V | Val |

Comme illustré plus loin, le demandeur a démontré l'efficacité de produits pour IRM comprenant un chélate lié au PEPTIDE MUC5AC notamment CPSIYPLLC.

Ainsi quand bien même un peptide serait connu de l'art antérieur, l'identification de son utilité dans un mécanisme de ciblage de MUC5AC, parmi le nombre extrêmement important de cibles biologiques possibles, est loin d'être évidente. De plus, il n'est nullement évident que cette cible biologique identifiée, des composés couplés (PEPTIDE MUC5AC-entité signal) permettent de résoudre les problèmes techniques résolus par le demandeur, notamment :
- la conservation de l'affinité en conditions physiologiques pour le site reconnaissance biologique, malgré l'encombrement stérique et la modification possible de conformation in vivo du fait du couplage à une entité signal
- la possibilité de couplage chimique avec les entités signal ;
- la stabilité physico-chimique *in vivo* qui constitue une limitation majeure pour de nombreux peptides
- la capacité d'être reconnu en imagerie en conditions physiologiques notamment *in vitro,* et cela en particulier en IRM, technique dont le niveau de sensibilité est près de 1000 fois inférieur à l'imagerie PET.

Au global, il n'était absolument pas évident pour l'homme du métier :
- d'une part de sélectionner les peptides objet de la présente invention
- d'autre part que les produits intégrant ces peptides soient en conditions physiologiques effectivement efficaces.

On décrit maintenant des exemples détaillés de réalisations de l'invention illustrée à l'aide des figures suivantes
Les figures 1, 2 et 3 illustrent le calcul de la valeur Kd* (constante apparente de dissociation) du peptide pour MUC5AC colique humain, MUC5AC gastrique murin (rat et souris) et pour MUC5AC directement après sécrétion sur cellules d'adénocarcinome humain sécrétrices respectivement.
La figure 4 montre des images de marquage anticorps anti-MUC5AC et MUC2, du peptide CPSIYPLLC (SEQ ID No2) sur coupes de tissus humains gastriques sain et d'adénomes coliques.

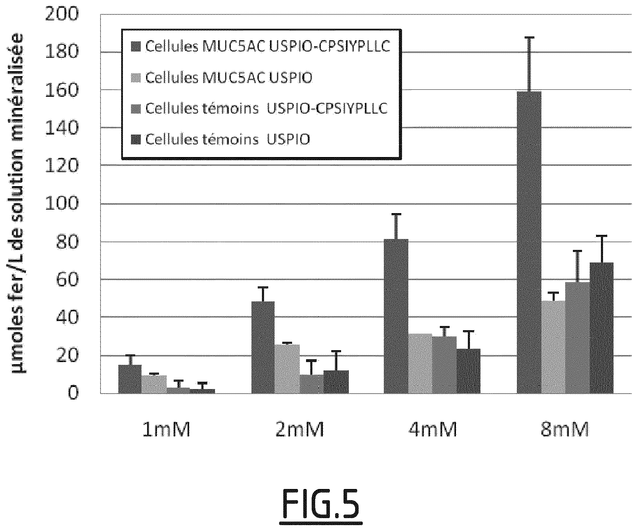

La figure 5 représente la quantité de fer directement corrélable à la fixation de l'USPIO-CPSIYPLLC et de l'USPIO seul sur deux lignées cellulaires d'adénocarcinome colique humain. Avec une lignée sécrétant du MUC5AC (cellules MUC5AC) et une lignée ne sécrétant pas de MUC5AC (cellules témoins).
Les figures 6A à D et 7A à D représentent la variation du signal en pourcentage de ΔSI (tumeur ou estomac ou foie ou rate / référence) en analyse RARE en fonction du temps en minutes de souris :
- porteuse d'une tumeur MUC5AC positive, signal dans la tumeur après injection de 20 µmol/kg de PEG750-C60c (n = 3) (figure 6A),
- porteuse d'une une tumeur MUC5AC positive, signal dans la tumeur après injection de 20 µmol/kg de composé contrôle (n = 5) (figure 6B),
- porteuse d'une une tumeur MUC5AC négative, signal dans la tumeur après injection de 20 µmol/kg de PEG750-C60c (n = 4) (figure 6C),
- porteuse d'une une tumeur MUC5AC positive, signal dans l'estomac après injection de 20 µmol/kg de composé contrôle (n = 2) (figure 6D),
- porteuse d'une tumeur MUC5AC positive, signal dans le foie après injection de 20 µmol/kg de PEG750-C60c (n = 3) (figure 7A),
- porteuse d'une une tumeur MUC5AC positive, signal dans le foie après injection de 20 µmol/kg de composé contrôle (n = 3) (figure 7B),
- porteuse d'une une tumeur MUC5AC négative, signal dans le foie après injection de 20 µmol/kg de PEG750-C60c (n = 2) (figure 7C),
- porteuse d'une une tumeur MUC5AC positive, signal dans la rate après injection de 20 µmol/kg de composé contrôle (n = 2) (figure 7D).

### EXEMPLES PARTIE I : préparation des composés incluant des PEPTIDES MUC5AC

### Généralités

M : concentration molaire (mole/l).
M/z : masse sur charge déterminé par spectrométrie de masse.
ES⁺ : électrospray mode positif.
ES⁻ : électrospray mode négatif.
kDa : unité de masse moléculaire (kiloDalton)
CCM : Chromatographie sur Couche Mince
Z ave : diamètre hydrodynamique mesuré par PCS

### Dosage du Fer total:

Le fer est dosé par spectroscopie d'absorption atomique (Spectrophotomètre VARIAN AA10) après minéralisation par HCl concentré et dilution par rapport à une gamme étalon d'ions ferrique (0, 5, 10, 15 et 20 ppm).

### Taille des particules :

### Diamètre hydrodynamique de la particule greffée (Z ave) :

Déterminé par PCS (appareil Malvern 4700, laser 488 nm à 90°) sur un échantillon dilué à ∼ 1 millimolaire avec de l'eau PPI filtrée sur 0.22 µm.

PCS = Photon Correlation Spectroscopy = Technique par Diffusion de Lumière Dynamique - Référence : R. Pecora dans J. of Nano. Res. (2000), 2, p 123-131.

### Analyses structurales :

Par spectroscopie de masse (appareil MICROMASS VG Quattro II) avec une source Electrospray.

### Mesures de relaxivité :

Les temps de relaxation T1 et T2 ont été déterminés par des procédures standards sur un appareil Minispec 120 (Bruker) à 20 Mhz (0,47T) et 37°C. Le temps de relaxation longitudinal T1 est mesuré en utilisant une séquence d'Inversion Récupération et le temps de relaxation transverse T2 est mesuré par une technique CPMG.
Les vitesses de relaxation R1 (=1/T1) et R2 (=1/T2) ont été calculées pour différentes concentrations en métal total (variant de 0,1.10⁻³ à 1.10⁻³ mole/L) en solution aqueuse à 37°C. La corrélation entre R1 ou R2 en fonction de la concentration est linéaire, et la pente représente la relaxivité r1 (R1/C) ou r2 (R2/C) exprimées en (1 / seconde) x (1/ mMole/L) soit (mM⁻¹.s⁻¹).

Les nanoparticules ont été préparées selon les méthodes décrites dans le brevet WO2004/058 275 (US 2004/253181) exemples 8 et 9 pour la préparation des solutions colloïdales de particules magnétiques et exemples 10 à 12 pour la complexation des particules magnétiques par un revêtement gembisphonate de l'exemple 1 de WO2004/058 275.

Les séquences peptidiques particulièrement d'intérêt sont présentées dans le tableau suivant :

| N° | code | peptide | séquence |
|---|---|---|---|
| 1 | C60 cyclique | 8-Amino-3,6-dioxaoctanoyl-cyclo-[Cys-Pro-Ser-Ile-Tyr-Pro-Leu-Leu-Cys]-NH2 | CPSIYPLLC |
| 2 | C60 | 8-Amino-3,6-dioxaoctanoyl-Pro-Ser-Ile-Tyr-Pro-Leu-Leu- NH2 | PSIYPLL |
| 3 | C16 | 8-Amino-3,6-dioxaoctanoyl-cyclo-[Cys-Ala-Leu-Ile-Pro-Pro-Leu-Leu-Cys]-NH2 | CALIPPLLC |

### Exemple I.1 : couplage des peptides sur la particule d'oxyde de fer

### Couplage du peptide N°1 sur une particule d'oxyde de fer

### Protocoles :

| N° | code | peptide | séquence |
|---|---|---|---|
| 1 | C60 cyclique | 8-Amino-3,6-dioxaoctanoyl-cyclo-[Cys-Pro-Ser-Ile-Tyr-Pro-Leu-Leu-Cys]-N H2 | CPSIYPLLC |

### Couplage :

15 ml de nanoparticules ([Fe]=0.338 M) sont agités à température ambiante, le pH est égal à 7.2. Une solution du peptide (N°1, 20mg) dan s 1ml d'eau est ajoutée par portion de 100µl avec 2.25mg d'EDCI toutes les 15 minutes. A la fin de l'addition, la solution est maintenue sous agitation 1 nuit. Le pH est ajusté à 7 avec une solution de NaOH 0,1M. La solution est ensuite Filtrée sur 0.22 µm (filtre Durapore Millipore®) et Ultrafiltrée sur une membrane de 30 kDa, puis concentrée jusqu'à un volume final de 15 ml.

### Couplage du PEG :

A 15 ml de la solution précédente est ajouté 2,5 ml d'une solution de 1,060g d'aminoPEG (O-(2-aminoethyl)-O'-methylpolyethyleneglycol 750, Aldrich, RN [80506-64-5]) dans 5 ml d'eau. Le pH de la solution est ajusté à 8 avec HCl 1M, puis 0,325g d'EDCI est ajouté et le mélange est agité 3h. L'ajout de la solution d'aminoPEG (2,5ml) et d'EDCI (0,325g) est renouvelé une fois et le mélange est agité à température ambiante une nuit. Le pH est ramené à 7.5 avec HCl 1M. La solution est filtrée sur 0.22 µm et ultrafiltrée sur membrane de 30 kDa. Le volume final de solution est de 15 ml.

### Caractérisation :

PCS : Z ave = 28 nm ; Concentration en fer :240 mM
r₁ (20MHz, 37°C) : 30.54 s⁻¹mM⁻¹
r₂ (20MHz, 37°C) : 96. s⁻¹mM⁻¹
r₁ (60MHz, 37°C) : 14.94 s⁻¹mM⁻¹
r₂ (60MHz, 37°C) : 95.06 s ⁻¹mM⁻¹

| Echantillons | Diamètre magne. | Aimantation à saturation (magn.). |
|---|---|---|
| PEG750-C60c | 7.2nm | 70Am²/Kg |

### Exemple I.2 : couplage des peptides sur un chélate de gadolinium (pour utilisation en IRM)

(on décrit l'utilisation de squarate mais il est clair que l'invention couvre tout linker tel que les groupes alkylène et/ou PEG cités dans la demande)

### Schéma général :

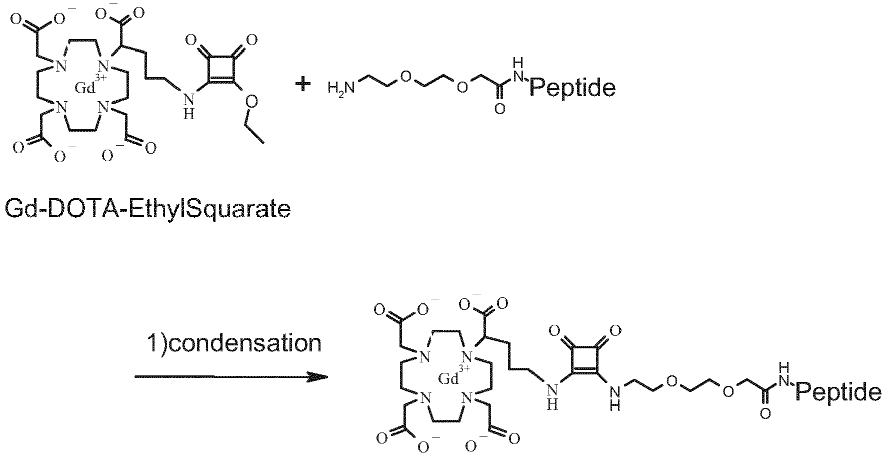

### séquence des peptides couplés :

| N° | code | peptide | séquence |
|---|---|---|---|
| 1 | C60 cyclique | 8-Amino-3,6-dioxaoctanoyl-cyclo-[Cys-Pro-Ser-Ile-Tyr-Pro-Leu-Leu-Cys]-NH2 | CPSIYPLLC |

### Condensation, déprotection :

200 mg de Gd-DOTA-diéthylsquarate (préparé selon WO2008125420) sont dissous dans 20 ml d'eau. le pH est ajusté à 9 avec une solution saturée de Na₂CO₃. 350 mg de peptide N°1 sont ajoutés et le pH est ajusté à 9.2. On laisse réagir 4 jours. La solution est dialysée sur des membranes de seuil de coupure1000 Da pendant 48h puis chromatographiée sur colonne RP-18 (Eluant : MeOH/eau (50/50)).

Le produit obtenu a pour structure :

| N° | Structure | PM | Spectro. de masse |
|---|---|---|---|
| 1 | 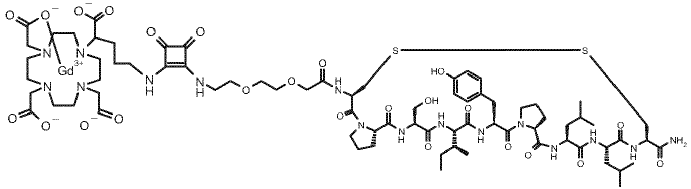 | 1843.20 | conforme |

### Exemple I.3 : couplage du peptide sur un chélate, le produit obtenu complexant le gallium (radionucléide pour imagerie TEP ; illustration avec un chélate de type NOTA)

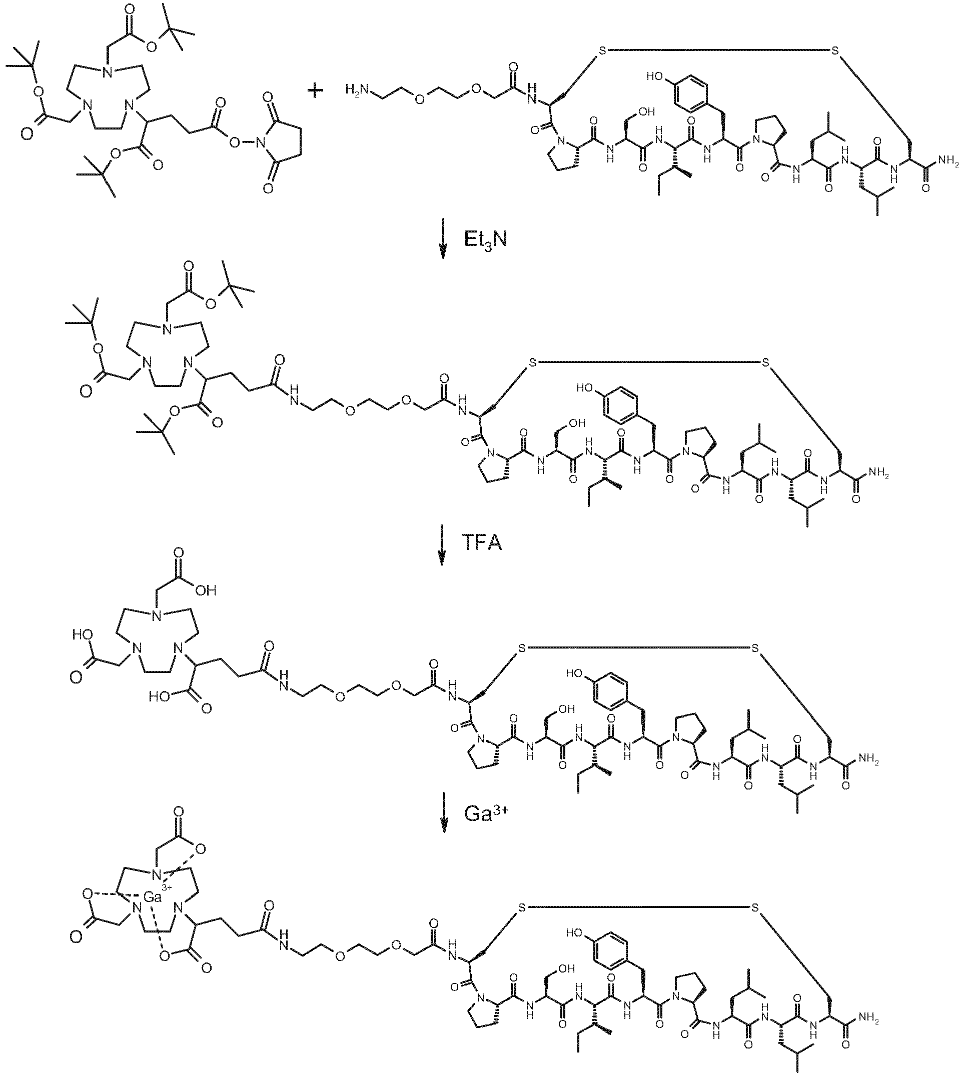

### 1- Formation de l'ester activé :

A 100mg (0.184 mmol) de NOTAGA(tBu)₃ (Chematech) dans 3 ml de CH₂Cl₂, sont ajoutés le NHS (1équivalent) puis le DCC (1 équivalent).Après ½ H de réaction à température ambiante, le DCU formé est filtré sur Whatman et le filtrat est concentré jusqu'à un volume final de 0.5 ml, environ.

### 2- Amidification :

Le peptide N°1 (1 équivalent) est dissous dans le DM SO en présence de 2éq de NEt₃: L'ester activé, en solution dans le CH₂Cl₂ est ajouté.

Après 2H de réaction le milieu réactionnel est précipité dans 25 ml d'Et₂O. Après filtration, lavage à l'Et₂O et séchage sous vide, on obtient des cristaux blancs. Rdt = 40%

### 3- Déprotection

Le produit ainsi obtenu est dissous dans le TFA. Après 7H de réaction à température ambiante, le TFA est évaporé. Le résidu obtenu est repris dans 4ml d'eau. Le produit est purifié sur cartouche de silice C18. 60mg de produit sont obtenus après lyophilisation

### 4- Marquage

20µg du composé précédemment préparé (20µl d'une solution à 1mg/ml) sont dissous dans 2ml de tampon acétate (PH 4) et 250mBq de Ga³⁺ issus du générateur sont ajoutés. Le milieu est agité à température ambiante durant 10min. puis purifié sur une cartouche C18 Sep-Pak®. La pureté radiochimique obtenue est de 95 à 99% (3essais).

### Exemple I.4 : couplage du peptide sur un colorant fluorescent

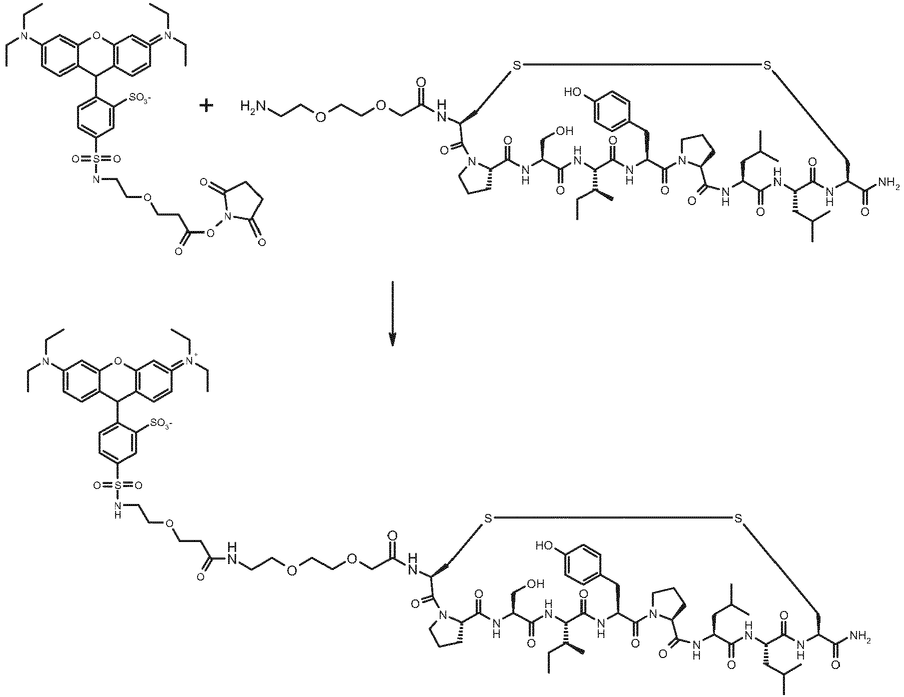

A une solution de 5mg de peptide et 10µl de triéthylamine dans 500µl de DMSO sont ajoutés 5 mg de colorant SRB-EOP-SE (FluoProbes ®). Le mélange est agité une nuit à température ambiante et à l'abri de la lumière. Le milieu réactionnel est précipité dans 20ml de diéthyléther. Le précipité est récupéré par centrifugation et rincé deux fois à l'éther. Après séchage, le produit est dissous dans l'eau et purifié par HPLC préparative sur une colonne C18 (10x250 mm) en utilisant un éluant (gradient) eau/Acétonitrile /TFA (0.1%). Après lyophilisation, le composé est stocké à -20°c. SM : ES⁻ = 1741

### EXEMPLES PARTIE II : caractérisation des produits

### Peptide CPSIYPLLC (SEQ ID No2)

### Mesure de la constante apparente de dissociation sur MUC5AC humaine

L'interaction entre le peptide et MUC5AC est évaluée à l'aide d'un test ELISA. La mucine MUC5AC (purifiée à partir de pièces tumorales humaines en s'appuyant sur des protocoles d'extraction et de purification de mucines décrits dans la littérature) est immobilisée sur plaque à la concentration 12.5µg/mL pendant toute une nuit à 4°C. Les sites non spécifiques sont ensuite saturés dans un tampon enrichi en lait pendant 2 heures à 4°C. Après plusieurs rinçages, le peptide CPSIYPLLC couplé à la biotine est incubé à des concentrations croissantes (10⁻³M et 10⁻⁶M) pendant 2 heures à 37°C. Les peptides biotinylés fixés à MUC5AC sont révélés à l'aide d'anticorps anti-biotine de chèvre incubés une heure à température ambiante puis dilués dans du PBS à 1 µg/mL. Après élimination des anticorps non liés, une seconde incubation d'une heure à température ambiante est réalisée en présence d'anticorps anti-chèvre marqués à la peroxydase à 0,2 µg/mL dilués dans du PBS complété par 0,1% de Tween-20. La réaction de coloration à la peroxydase est réalisée en présence d'ABTS enrichi en H₂O₂. La mesure de la DO à 405nm permet le calcul de la valeur K*_{d} MUC5AC.
L'interaction entre le peptide et MUC5AC est également évaluée à l'aide du test ELISA décrit précédemment sur la mucine MUC5AC préalablement purifiée à partir d'estomac de souris et de rat.

L'affinité du peptide pour MUC5AC est calculée à partir d'un test fonctionnel cellulaire qui consiste à mesurer la fixation du peptide sur un modèle cellulaire sécrétant MUC5AC. Après 21 jours de culture pour les cellules HT29-5M21 et 7 jours pour les cellules HCT116, les cellules sont rincées avec du PBS à 4°C, puis fixées avec 1% de glutaraldehyde dilué dans du PBS. Les cellules sont ensuite rincées à nouveau 3 fois avec du PBS à 4°C et incubées 2H à température ambi ante avec un tampon enrichi en lait et 2H à 37°C dans une gamme de concentration comprise entre 10⁻³M et 10⁻⁶M de peptides biotinylés. Les peptides biotinylés fixés à la cible sont révélés à l'aide d'anticorps anti-biotine de chèvre incubés une heure à température ambiante (dilués dans du PBS à 1 µg/mL). Une seconde incubation d'une heure à température ambiante est réalisée en présence d'anticorps anti-chèvre marqués à la peroxydase à 0,2 µg/mL dilués dans du PBS complété par 0,1% de Tween-20. La réaction de coloration à la peroxydase est réalisée en présence d'ABTS et la mesure de la DO est réalisée à 405nm. Le test cellulaire a l'intérêt de montrer des données d'affinité, sa sensibilité étant encore perfectible. La sécrétion de MUC5AC a été bien vérifiée dans l'étude. Les résultats sur tissus humains (courbe d'affinité pour la MUC5AC humaine obtenue également) donnent des résultats très positifs.

### Evaluation de la spécificité sur coupes de tissus

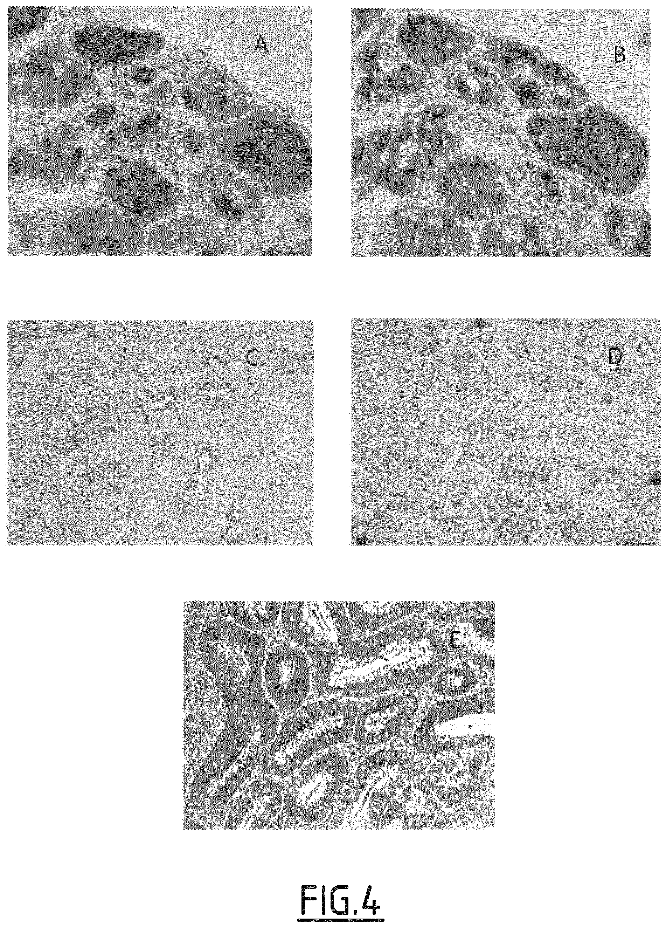

Des coupes de muqueuses gastrique, colique saine et d'adénome colorectal humain (5 µm d'épaisseur) ont été fixées 24h par du paraformaldéhyde 4% et incluses dans de la paraffine. Pour le marquage, les coupes sont tout d'abord déparaffinées dans du toluène et réhydratées dans de l'alcool 95%. Après rinçages dans de l'eau, les lames sont portées à ébullition durant 10 minutes dans un tampon citrate (50 mM, pH 4). Une fois la température du tampon revenue à 25-30°C, les lames sont ensuite rincées au PBS puis successivement incubées durant 15 minutes dans une solution de peroxyde d'hydrogène (H₂O₂) 0.15% et un kit de blocage de la biotine endogène pendant une 1 h. Puis les coupes sont bloquées à l'aide d'un tampon enrichi en lait, 1h à température ambiante, pour être ensuite incubées à 4°C, 18h avec les peptides à différentes concentrations (20 µM, 10 µM, 1 µM, 0.2 µM, 0.04 µM) ou les anticorps (MUC5AC : PM8 à la dilution 1/100 et MUC2 : Lum2-3 à la dilution 1/100) dilués dans du PBS. Les peptides biotinylés fixés à la cible sont révélés à l'aide d'anticorps anti-biotine de chèvre incubés une heure à température ambiante puis dilués dans du PBS à 1 µg/mL. Une seconde incubation d'une heure à température ambiante est réalisée en présence d'anticorps anti-chèvre marqués à la peroxydase à 0,2 µg/mL dilués dans du PBS complété par 0,1% de Tween-20. Les anticorps anti-MUC5AC et anti-MUC2 sont révélés à l'aide d'un anticorps secondaire anti-souris et anti-lapin, respectivement, couplés à la peroxydase et dilués chacun au 1/250. Après différents lavages dans du PBS les lames sont incubées avec du DAB (3,3'-Diaminobenzidine tetrahydrochloride) 0,05%, complété avec 0,015% d'H₂O₂ durant 50 minutes. Les différentes coupes utilisées (figure 4) sont de l'épithélium gastrique humain sain marqué avec le peptide CPSIYPLLC biotinylé (grossissement X50) (A), avec des anticorps monoclonaux anti-MUC5AC, (PM8, grossissement X50) (B). Mais également de l'adénome colique marqué à l'aide du peptide CPSIYPLLC (grossissement X10), sur tissus coupés de façon transversale (C), avec des anticorps monoclonaux anti-MUC5AC, (PM8, grossissement X10), sur tissus coupés de façon transversale (D), et à l'aide d'anticorps polyclonaux anti-MUC2 (grossissement X10), sur tissus coupés de façon transversale (E).

### Produit de contraste USPIO- CPSIYPLLC

### Test sur modèles cellulaire coliques

Après 21 jours de culture pour les cellules HT29-5M21 (cellules MUC5AC) la littérature rapporte que MUC5AC est massivement exprimé. Les cellules HCT116 n'expriment pas MUC5AC (cellules témoins) les cellules sont rincées avec du PBS à 4°C, puis directement mises en contact avec 0.5mL d'USPIO dilués dans du PBS durant 1H 37°C (incubateur 5% de CO₂). Les USPIO sont testés à différentes concentrations de 1/2/4/8 mM en fer sur chacune des lignées. Après aspiration du surnageant, les cellules sont lavées deux fois avec du PBS puis grattées pour ensuite être reprises par 250µL de PBS (2 fois). Les suspensions sont ensuite lyophilisées puis reprises par 100µL d'une solution d'HCl à 5N 4H à 80°C au bain-marie. Le dosage est ensuite réal isé à l'aide du réactif de Perl's avant de réaliser la relaxométrie (Bruker Minispec 60 MHz). La sécrétion de MUC5AC par le modèle HT29 et l'absence de sécrétion avec HCT116 ont été vérifiées dans l'étude.

### Références

Gouyer V, Wiede A, Buisine MP, Dekeyser S, Moreau O, Lesuffleur T, Hoffmann W and Huet G (2001) Specific secretion of gel-forming mucins and TFF peptides in HT-29 cells of mucin-secreting phenotyp, Biochim. Biophys. Acta. 1539 : 71-84.
Sheehan JK, Brazeau C, Kutay S, Pigeaon H, Kirkham S, Howard M and Thornton DJ (2000) Physical characterization of the MUC5AC mucin: a highly oligomeric glycoprotein whether isolated from cell culture or in vivo from respiratory mucous secretions, Biochem. Journal. 347 : 37-44.

### EXEMPLES PARTIE III : tests biologiques

### III.1 Peptide CPSIYPLLC (SEQ ID No2)

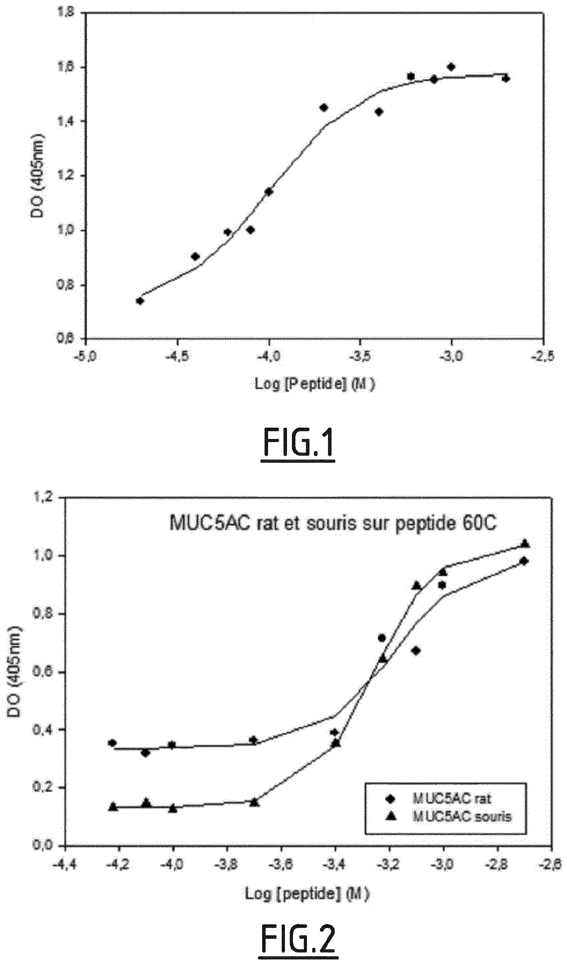

Le test ELISA (figure 1) basé sur l'immobilisation de MUC5AC purifié en présence de concentrations croissantes du peptide CPSIYPLLC permet de calculer une valeur K*_{d} constante de dissociation apparente égale à 8,5.10⁻⁵ M, ce qui démontre la bonne interaction entre le peptide et la molécule MUC5AC humaine. Les valeurs K*_{d} constante de dissociation apparente sont de 6,59.10⁻⁴M et de 5,49.10⁻⁴M entre le peptide CPSIYPLLC et respectivement la glycoprotéine MUC5AC de rat et de souris (figure 2), ceci indique que les modèles murins ou rat sont des modèles pertinents pour prédire la liaison du produit de contraste USPIO- CPSIYPLLC à la cible MUC5AC.

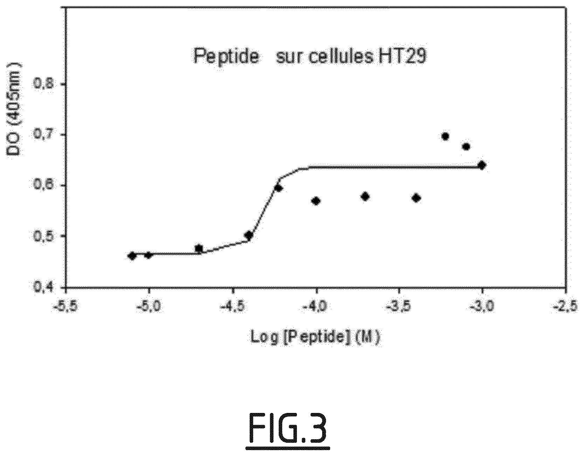

Le test de fixation sur cellules cancéreuses coliques humaines (HT29 5M21) du peptide CPSIYPLLC (figure 3) permet de calculer une valeur K*_{d} constante de dissociation apparente égale à 4,8.10⁻⁵ M, ce qui témoigne de l'interaction spécifique entre le peptide et MUC5AC natif sur cellules vivantes.

Le marquage de coupes d'épithéliums gastriques humain sains (figure 4) permet de démontrer la spécificité du peptide, tout comme le marquage de coupes d'adénome colique qui indiquent également l'absence de reconnaissance par le peptide CPSIYPLLC de la glycoprotéine MUC2 qui est la mucine majoritaire de l'intestin.

### III.2 Produit de contraste USPIO- CPSIYPLLC

La concentration en fer augmente significativement sur les cellules MUC5AC en présence de concentration croissante d'USPIO-CPSIYPLLC. A l'inverse, en présence de ce même produit sur les cellules témoins, la concentration n'évolue pas proportionnellement à la concentration d'USPIO-CPSIYPLLC. De façon comparable, l'USPIO sans le peptide que ce soit sur les cellules MUC5AC ou sur les cellules HCT116 (MUC5AC-négatives) n'influence pas la concentration en fer. On peut donc en déduire que l'USPIO-CPSIYPLLC est capable de se fixer sur les cellules MUC5AC. Ces résultats démontrent que le greffage du peptide CPSIYPLLC (SEQ ID No2) sur l'USPIO permet de cibler spécifiquement MUC5AC sécrétée par des cellules humaines d'adénocarcinome colique, avec un effet dose. Il y a ciblage spécifique de MUC5AC, la spécificité étant observée pour les concentrations les plus fortes, en particulier 4 et 8 mM en fer.

### EXEMPLES PARTIE IV : imagerie

Dans les expériences qui suivent, les deux composés suivants ont été utilisés :
1) Composé selon l'invention : PEG750-C60c de l'exemple 1.1. ci-dessus (peptide CPSIYPLLC couplé sur une particule de fer)
2) Composé contrôle, qui ne comprend pas le peptide ciblant MUC5A et a été préparé comme suit :
   a) préparation des solutions colloïdales de particules magnétiques selon les méthodes décrites dans les exemples 8 et 9 de la demande WO2004/058 275, puis
   b) complexation des particules magnétiques par un revêtement gembisphonate de l'exemple 1 de WO2004/058 275 selon les méthodes des exemples 10 à 12 de la demande WO2004/058 275, puis
   b) couplage avec le PEG 750 suivant le protocole suivant :
      A15 ml de nanoparticules ([Fe]=0.338 M) agitées à température ambiante ont été ajoutés 2,5 ml d'une solution de 1,060g d'aminoPEG (O-(2-aminoethyl)-O'-methylpolyethyleneglycol 750, Aldrich, RN [80506-64-5]) dans 5 ml d'eau. Le pH de la solution a été ajusté à 8 avec HCl 1M, puis 0,325g d'EDCI ont été ajoutés et le mélange a été agité 3h. L'ajout de la solution d'aminoPEG (2,5ml) et d'EDCI (0,325g) a été renouvelé une fois et le mélange a été agité à température ambiante une nuit. Le pH a été ramené à 7.5 avec HCl 1M. La solution a été filtrée sur 0.22 µm et ultrafiltrée sur membrane de 30 kDa. Le volume final de solution était de 15 ml.

### IV.1 Imagerie par résonance magnétique (IRM) moléculaire

Les paramètres d'imagerie sélectionné sont les suivants :
Séquence RARE (rapid acquisition with relaxation enhancement) (7T)
TR/TEeff = 3000 / 48.7 ms,
facteur RARE = 4,
NEX = 4,
Matrice = 256x256
FOV = 2.3 cm
épaisseur de coupe = 1 mm
20 coupes axiales
résolution spatiale = 90 µm
Temps d'acquisition = 12 min 48 s

Des souris :
- porteuses d'une tumeur MUC5AC positive ont été injectées avec PEG750-C60c (100 µmol/kg),
- porteuses d'une tumeur MUC5AC positive ont été injectées avec le composé contrôle (100 µmol/kg) et
- porteuses d'une tumeur MUC5AC négative ont été injectées avec PEG750-C60c (100 µmol/kg).

Les résultats suivants ont été observés sur les images d'IRM des tumeurs:
- Les images d'IRM pré et post contraste de la tumeur MUC5AC positive traitée avec PEG750-C60c apparaissent plus sombre et a donc observé une image en contraste négatif après injection de PEG750-C60c avec une faible intensité de pixels.
- A l'inverse, la tumeur MUC5AC positive traitée avec le composé contrôle apparait plus claire et a donc une image en contraste positif avec une forte intensité de pixels.
- L'effet observé dans la tumeur MUC5AC positive traitée avec PEG750-C60c n'est pas retrouvé chez la souris porteuse d'une tumeur MUC5AC négative traitée avec PEG750-C60c, puisque l'on n'observe pas de variation de contraste dans la tumeur.

L'estomac a également été imagé puisqu'il possède naturellement chez l'Homme et les rongeurs de la mucine MUC5AC. On a pu observer après injection de PEG750-C60c chez les souris porteuses d'une tumeur MUC5AC positive ou négative une zone gastrique en contact avec la lumière qui présente un net assombrissement délimité par le tissu supérieur gastrique. Ce résultat démontre clairement la validité de PEG750-C60c pour cibler la mucine MUC5AC. En effet, en utilisant PEG750-C60c à concentration égale chez des animaux ayant des tumeurs au profil d'expression de MUC5AC, opposé, mais possédant la même caractéristique de sécrétion de mucines dans l'estomac, nous avons observé clairement une réponse différentielle dans la tumeur, mais pas dans l'estomac.

Les expériences imagerie IRM sur les animaux porteurs de xénogreffes sont résumées dans le tableau 2.

**Tableau 2 : Récapitulatif des nombres de souris imagées dans la tumeur au cours des deux campagnes d'imagerie.**

| | 100µmol/kg | 20µmol/kg |
|---|---|---|
| MUC5AC+ PEG750-C60c | 4 | 3 |
| MUC5AC+ Composé contrôle | 5 | 5 |
| MUC5AC- PEG750-C60c | 2 | 4 |

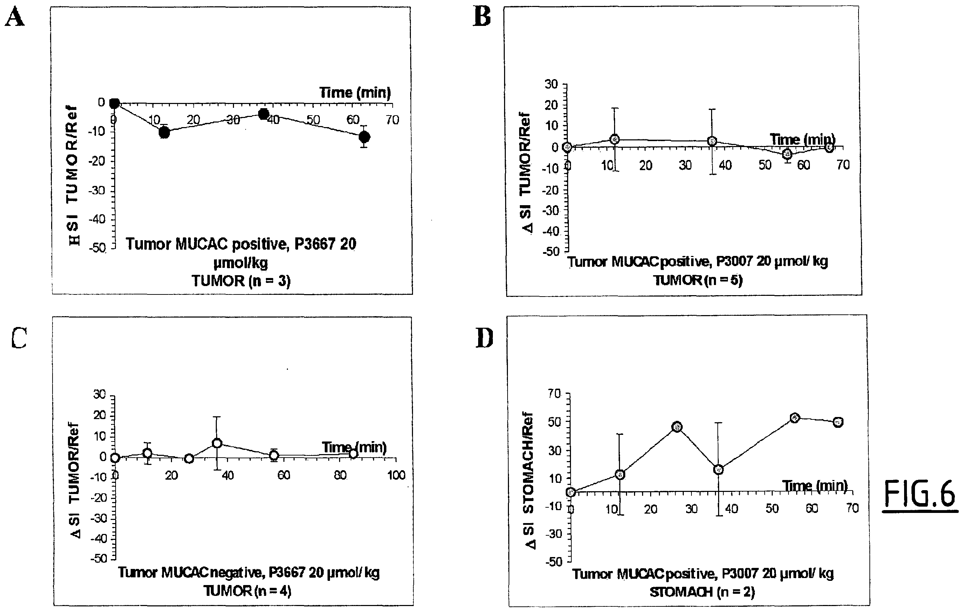

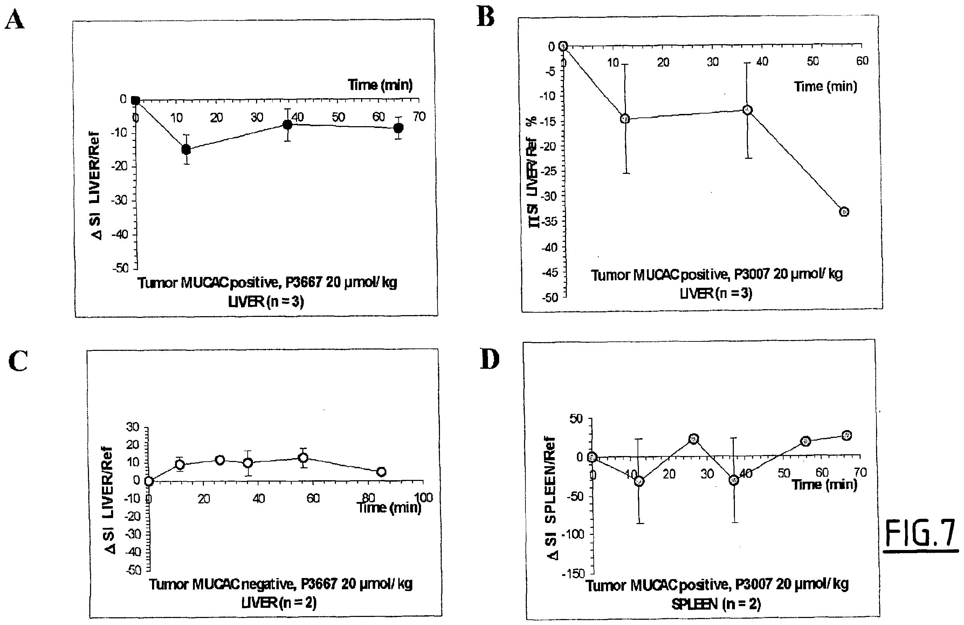

Quelle que soit la dose de PEG750-C60c utilisée, les tumeurs MUC5AC positives présentent un signal reproductible. Le signal décroit entre -10% (post-contraste) et -15% à respectivement 20µmol de fer/kg et 100µmol de fer/kg (Figures 6 et 7). Dans les mêmes conditions, les deux contrôles négatifs (PEG750-C60c/MUC5AC négatif et Composé contrôle /MUC5AC positif) voient leur signal dans la tumeur ne pas évoluer ou augmenter légèrement.
On constate dans l'estomac après injection de PEG750-C60c à 100µmol/kg, qu'il y a une décroissance de signal importante comprise entre -40% et -45%. L'injection du composé contrôle abaisse le signal dans l'estomac de -15%.

### IV.2 Cinétique plasmatique : calcul de la concentration en USPIO contenue dans le plasma des souris imagées

On peut observer dans le tableau 3 que les concentrations en fer sont proportionnelles à la dose de produit injectée. De plus, les concentrations déterminées suggèrent que les produits ne sont que très peu éliminés 1h-1h30 après leur injection.

**Tableau 3 : Concentration en fer mesurée par relaxométrie dans le plasma des souris.**

| Souris | Concentration en fer (µmol/L) |
|---|---|
| 1 MUC5AC positive PEG750-C60c 20 µmol Fe/kg | 380 |
| 2 MUC5AC positive composé contrôle 20 µmol Fe/kg | 411,5 |
| 3 MUC5AC négative PEG750-C60c 20 µmol Fe/kg | 471,5 |
| 4 MUC5AC positive PEG750-C60c 40 µmol Fe/kg | 909,6 |
| 6 MUC5AC positive PEG750-C60c 20 µmol Fe/kg | 486,8 |
| 7 MUC5AC positive PEG750-C60c 100 µmol Fe/kg | 1555,5 |
| 8 MUC5AC positive composé contrôle 100 µmol Fe/kg | 1804,8 |
| 9 MUC5AC positive composé contrôle 20 µmol Fe/kg | 306,4 |
| 10 MUC5AC positive PEG750-C60C 20 µmol Fe/kg | 312,4 |
| 11 MUC5AC négative PEG750-C60C 20 µmol Fe/kg | 442,5 |
| 12 MUC5AC positive composé contrôle 20 µmol Fe/kg | 334,4 |
| 13 MUC5AC positive composé contrôle 20 µmol Fe/kg | 343,3 |
| 14 MUC5AC négative PEG750-C60C 20 µmol Fe/kg | 350,7 |
| 1 MUC5AC positive PEG750-C60C 100 µmol Fe/kg | 2590 |
| 2 MUC5AC positive composé contrôle 100 µmol Fe/kg | 1883 |
| 3 MUC5AC positive PEG750-C60C 100 µmol Fe/kg | 3871 |
| 4 MUC5AC positive composé contrôle 100 µmol Fe/kg | 1667 |
| 5 MUC5AC positive PEG750-C60C 100 µmol Fe/kg | 1959 |
| 6 MUC5AC positive composé contrôle 100 µmol Fe/kg | 1781 |
| 7 MUC5AC positive composé contrôle 100 µmol Fe/kg | 1908 |
| 8 MUC5AC positive composé contrôle 20 µmol Fe/kg | 182 |
| 9 MUC5AC négative PEG750-C60C 20 µmol Fe/kg | 337 |
| 10 MUC5AC négative PEG750-C60C 100 µmol Fe/kg | 2806 |
| 11 MUC5AC négative PEG750-C60C 100 µmol Fe/kg | 3638 |
| 12 MUC5AC positive composé contrôle 100 µmol Fe/kg | 2082 |

### IV.3 Corrélation entre les résultats d'imagerie et les modèles animaux

On peut constater dans le tableau 4, après analyse anatomopathologique, que toutes les tumeurs ont un profil d'expression de MUC5AC attendu. En effet, les xénogreffes réalisées avec la lignée sécrétant MUC5AC (HT29 5M21) expriment toujours la mucine MUC5AC de façon constante et la lignée ne la sécrétant pas ne l'exprime jamais. Ces résultats permettent de valider les résultats observés par analyse IRM.

**Tableau 4 : Animaux utilisés et profil d'expression de MUC5AC par histologie.**

| Animaux | Produit injecté | Dose (µmol/kg) | Cellules | Immuno MUC5AC |
|---|---|---|---|---|
| Souris 1 | PEG750-C60C | 20 | HT29 5M21 | + |
| Souris 2 | composé contrôle | 20 | HT29 5M21 | + |
| Souris 3 | PEG750-C60C | 20 | HCT116 | - |
| Souris 4 | PEG750-C60C | 40 | HT29 5M21 | + |
| Souris 6 | PEG750-C60C | 20 | HT29 5M21 | + |
| Souris 7 | PEG750-C60C | 100 | HT29 5M21 | + |
| Souris 8 | composé contrôle | 100 | HT29 5M21 | + |
| Souris 9 | composé contrôle | 20 | HT29 5M21 | + |
| Souris 10 | PEG750-C60C | 20 | HT29 5M21 | + |
| Souris 11 | PEG750-C60C | 20 | HCT116 | - |
| Souris 12 | composé contrôle | 20 | HT29 5M21 | + |
| Souris 13 | composé contrôle | 20 | HT29 5M21 | + |
| Souris 14 | PEG750-C60C | 20 | HCT116 | - |
| Souris 1 | PEG750-C60C | 100 | HT29 5M21 | + |
| Souris 2 | composé contrôle | 100 | HT29 5M21 | + |
| Souris 3 | PEG750-C60C | 100 | HT29 5M21 | + |
| Souris 4 | composé contrôle | 100 | HT29 5M21 | + |
| Souris 5 | PEG750-C60C | 100 | HT29 5M21 | + |
| Souris 6 | composé contrôle | 100 | HT29 5M21 | + |
| Souris 7 | composé contrôle | 100 | HT29 5M21 | + |
| Souris 8 | composé contrôle | 20 | HT29 5M21 | + |
| Souris 9 | PEG750-C60C | 20 | HCT116 | - |
| Souris 10 | PEG750-C60C | 100 | HCT116 | - |
| Souris 11 | PEG750-C60C | 100 | HCT116 | - |
| Souris 12 | composé contrôle | 100 | HT29 5M21 | + |

### IV.4 Détection des USPIO sur tissus après injection in vivo chez l'animal

On a pu constater, dans les tumeurs, une accumulation du PEG750-C60C uniquement lorsqu'elle sécrète MUC5AC. De plus, on a pu remarquer un marquage spécifique, donc une accumulation du PEG750-C60C dans le tissu gastrique de souris et une absence de marquage chez les animaux traités avec le composé contrôle.

Dans les expériences réalisées, le PEG750-C60C a été détecté à l'aide de l'anticorps anti-PEG sur xénogreffe HT295M21, sur xénogreffe HCT116 et sur coupe d'estomac de souris sans contre coloration. Le composé contrôle a été détecté sur coupe d'estomac de souris sans contre coloration. Les coupes ont été parallèlement révélées avec un anticorps anti-MUC5AC sur HT295M21 et HCT116.

Ces résultats permettent de valider l'analyse imagerie et de confirmer les images obtenues en IRM dans l'estomac de souris injectées avec le PEG750-C60C. En effet, on peut observer une apparition de signal dans une couche indirectement en contact avec la lumière gastrique chez les animaux traités. On peut donc en conclure que le produit ne s'accumule pas jusqu'à la partie supérieure de l'épithélium gastrique, là où, pourtant, MUC5AC se trouve également. Ceci peut s'expliquer par l'impossibilité de l'USPIO à diffuser jusqu'à la surface, à sa détérioration au pH acide de l'estomac ou encore une captation par MUC5AC présent dans le bas des cryptes gastriques empêchant ainsi la progression des USPIO.

## Revendications

1. Composé de formule générale (I) suivante :
Signal-Lien-Peptide (I)
Dans laquelle :
- Signal représente une entité signal ;
- Lien, présent ou non, représente une partie de liaison chimique et
- Peptide représente un peptide comprenant un peptide ciblant MUC5AC, le peptide ciblant MUC5AC étant choisi parmi les peptides de formule suivante :
a) X1-X2-X3-X4-X5-X6-X7-X8-X9 (1) (SEQ ID No1) avec:
- X1 est absent ou choisi parmi la cystéine, la méthionine
- X2 choisi parmi l'hydroxyproline ou la proline
- X3 choisi parmi la thréonine ou la sérine
- X4 choisi parmi l'isoleucine, la leucine, la valine
- X5 choisi parmi la tyrosine, la phénylalanine, le tryptophane
- X6 choisi parmi l'hydroxyproline ou la proline
- X7 choisi parmi l'isoleucine, la leucine, la valine
- X8 choisi parmi l'isoleucine, la leucine, la valine
- X9 est absent ou choisi parmi la cystéine, la méthionine ;
b) X10-X11-X12-X13-X14-X15-X16-X17-X18 (2) (SEQ ID No4) avec:
- X10 est absent ou choisi parmi la cystéine, la méthionine
- X11 choisi parmi l'isoleucine, la leucine, la valine, alanine
- X12 choisi parmi la thréonine ou la sérine
- X13 choisi parmi l'isoleucine, la leucine, la valine
- X14 choisi parmi l'hydroxyproline ou la proline
- X15 choisi parmi l'hydroxyproline ou la proline
- X16 choisi parmi l'isoleucine, la leucine, la valine
- X17 choisi parmi l'isoleucine, la leucine, la valine
- X18 est absent ou choisi parmi la cystéine, la méthionine;
c) CALIPPLLC (SEQ ID No5)
les sels pharmaceutiquement acceptables de ces composés du a) ou du b).

2. Composé selon la revendication 1 dans laquelle Peptide est un peptide de formule CPSIYPLLC (SEQ ID No2), peptide PSIYPLL (SEQ ID No3) ou peptide CALIPPLLC (SEQ ID No5).

3. Composé selon la revendication 1 ou 2 dans laquelle Signal est un chélate couplé ou non à un métal paramagnétique M, M étant choisi avantageusement parmi Gd, Mn, Fe, Dy et Tm.

4. Composé selon la revendication 1 ou 2 dans laquelle Signal est un chélate couplé ou non à un radionucléide ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho.

5. Composé selon la revendications 3 ou 4 dans laquelle le chélate est choisi parmi : DOTA, DTPA, DO3A, HPDO3A, BOPTA, NOTA, PCTA, DOTMA, AAZTA, HOPO et leurs dérivés

6. Composé selon l'une quelconque des revendications 1 à 5 dans laquelle Lien représente :
a) un groupe de formule : Q1-I-Q2,
dans laquelle Q1 et Q2 identiques ou différents représentent O, S, NH, CO₂, - NHCO, CONH, NHCONH, NHCSNH, SO₂NH- ou NHSO₂-,
et I représente un groupe alkyle, alkoxyalkyle, polyalkoxyalkylène, alcényle, alcynyle, alkyle interrompu par un ou plusieurs squarate, par un ou plusieurs aryles, avantageusement phényle, ou par un ou plusieurs groupes choisis parmi -NH-, -O-, -CO-, -NH(CO)-, -(CO)NH-, -O(CO)-, ou -(OC)O- ;
b) un groupe (CH₂)ₙ, (CH₂)ₙ-CO-, -(CH₂)ₙNH-CO- avec n = 2 à 10, (CH₂CH₂O)_{q}(CH₂)ᵣ-CO-, (CH₂CH₂O)q(CH₂)ᵣ-NH-CO- avec q = 1-10 et r=2-10, (CH₂)ₙ-CONH-, (CH₂)ₙ-CONH-PEG, (CH₂)ₙ-NH-, squarate, avec n=1 à 5 et avantageusement n=4 ou 5, HOOC-CH₂-O-(CH₂)₂-O-(CH₂)₂-O-CH₂-COOH ; HOOC-(CH₂)₂-CO₂-(CH₂)₂-OCO-(CH₂)₂-COOH ; HOOC-CH(OH)-CH(OH)-COOH; HOOC-(CH₂)ₙ-COOH; NH₂-(CH₂)ₙ-NH₂, avec n=1-20; NH₂-(CH₂)ₙ-CO₂H ; ou NH₂-CH₂-(CH₂-O-CH₂)ₙ-CO₂H avec n=1 à 10.

7. Composé selon la revendication 1 ou 2 dans laquelle Signal représente une nanoparticule superparamagnétique revêtue d'une couche organique, avantageusement comportant un noyau d'oxyde de fer.

8. Composé selon la revendication 7 dans laquelle la couche organique est une couche gem-bisphosphonate.

9. Composé selon la revendication 1 ou 2 dans laquelle Signal représente un marqueur pour imagerie optique.

10. Composé selon la revendication 1 ou 2 dans laquelle Signal représente une nanoparticule lipidique comprenant au moins un chélate, avantageusement le chélate est tel que défini dans la revendication 5.

11. Composition pour imagerie médicale comprenant au moins un composé de formule générale (I) selon l'une des revendications 1 à 10 et un excipient pharmaceutiquement acceptable.

12. Composé (I) selon l'une des revendications 1 à 10 destiné à être utilisé dans une méthode de diagnostique d'une pathologie associée à MUC5AC, notamment choisie parmi les maladies héréditaires colorectales, les maladies inflammatoires chroniques du tube digestif, les populations risquant de contracter un cancer colorectal, les suivis de récidives chez les patients ayant déjà été traités du cancer colorectal.

13. Utilisation d'un composé (I) selon l'une des revendications 1 à 10 pour la préparation d'une composition de diagnostique d'une pathologie associée à MUC5AC, notamment choisie parmi les maladies héréditaires colorectales, les maladies inflammatoires chroniques du tube digestif, les populations risquant de contracter un cancer colorectal, les suivis de récidives chez les patients ayant déjà été traités du cancer colorectal.

## Patentansprüche

1. Verbindung der folgenden allgemeinen Formel (I):
Signal-Linker-Peptid (I),
worin:
- Signal eine Signaleinheit darstellt,
- Linker, der vorhanden ist oder nicht, ein chemisches Verknüpfungsteil darstellt und
- Peptid ein Peptid darstellt, das ein auf MUC5AC abzielendes Peptid umfasst, wobei das auf MUC5AC abzielende Peptid aus den Peptiden der folgenden Formel ausgewählt ist:
a) X1-X2-X3-X4-X5-X6-X7-X8-X9 (1) (SEQ ID Nr. 1), wobei:
- X1 fehlt oder aus Cystein, Methionin ausgewählt ist,
- X2 aus Hydroxyprolin oder Prolin ausgewählt ist,
- X3 aus Threonin oder Serin ausgewählt ist,
- X4 aus Isoleucin, Leucin, Valin ausgewählt ist,
- X5 aus Tyrosin, Phenylalanin, Tryptophan ausgewählt ist,
- X6 aus Hydroxyprolin oder Prolin ausgewählt ist,
- X7 aus Isoleucin, Leucin, Valin ausgewählt ist,
- X8 aus Isoleucin, Leucin, Valin ausgewählt ist,
- X9 fehlt oder aus Cystein, Methionin ausgewählt ist;
b) X10-X11-X12-X13-X14-X15-X16-X17-X18 (2) (SEQ ID Nr. 4), wobei:
- X10 fehlt oder aus Cystein, Methionin ausgewählt ist,
- X11 aus Isoleucin, Leucin, Valin, Alanin ausgewählt ist,
- X12 aus Threonin oder Serin ausgewählt ist,
- X13 aus Isoleucin, Leucin, Valin ausgewählt ist,
- X14 aus Hydroxyprolin oder Prolin ausgewählt ist,
- X15 aus Hydroxyprolin oder Prolin ausgewählt ist,
- X16 aus Isoleucin, Leucin, Valin ausgewählt ist,
- X17 aus Isoleucin, Leucin, Valin ausgewählt ist,
- X18 fehlt oder aus Cystein, Methionin ausgewählt ist;
c) CALIPPLLC (SEQ ID Nr. 5)
den pharmazeutisch annehmbaren Salzen dieser Verbindungen unter a) oder b).

2. Verbindung nach Anspruch 1, worin Peptid ein Peptid der Formel CPSIYPLLC (SEQ ID Nr. 2), Peptid PSIYPLL (SEQ ID Nr. 3) oder Peptid CALIPPLLC (SEQ ID Nr. 5) ist.

3. Verbindung nach Anspruch 1 oder 2, worin Signal ein Chelat ist, das an ein paramagnetisches Metall M gekoppelt ist oder nicht, wobei M vorteilhafterweise aus Gd, Mn, Fe, Dy und Tm ausgewählt ist.

4. Verbindung nach Anspruch 1 oder 2, worin Signal ein Chelat ist, das an ein Radionuklid ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho gekoppelt ist oder nicht.

5. Verbindung nach den Ansprüchen 3 oder 4, wobei das Chelat aus Folgenden ausgewählt ist: DOTA, DTPA, DO3A, HPDO3A, BOPTA, NOTA, PCTA, DOTMA, AAZTA, HOPO und deren Derivaten.

6. Verbindung nach einem der Ansprüche 1 bis 5, worin Linker für Folgendes steht:
a) eine Gruppe der Formel: Q1-I-Q2,
worin Q1 und Q2, die gleich oder verschieden sind, für O, S, NH, CO₂, - NHCO, CONH, NHCONH, NHCSNH, SO₂NH- oder NHSO₂- stehen
und I eine Alkyl-, Alkoxyalkyl-, Polyalkoxyalkylen-, Alkenyl-, Alkinyl-, Alkylgruppe darstellt, die durch ein oder mehrere Quadratate, durch ein oder mehrere Aryle, vorteilhafterweise Phenyl, oder durch eine oder mehrere Gruppen, ausgewählt aus -NH-, -O-, -CO-, -NH(CO)-, -(CO)NH-, -O(CO)- oder -(OC)O-, unterbrochen ist;
b) eine Gruppe (CH₂)ₙ, (CH₂)ₙ-CO-, -(CH₂)ₙNH-CO- mit n = 2 bis 10, (CH₂CH₂O)_{q}(CH₂)ᵣ-CO-, (CH₂CH₂O)_{q}(CH₂)ᵣ-NH-CO- mit q = 1-10 und r = 2-10, (CH₂)ₙ-CONH-, (CH₂)ₙ-CONH-PEG, (CH₂)ₙ-NH-, Quadratat, mit n = 1 bis 5 und vorteilhafterweise n = 4 oder 5, HOOC-CH₂-O-(CH₂)₂-O-(CH₂)₂-O-CH₂-COOH; HOOC-(CH₂)₂-CO₂-(CH₂)₂-OCO-(CH₂)₂-COOH; HOOC-CH(OH)-CH(OH)-COOH; HOOC-(CH₂)ₙ-COOH; NH₂-(CH₂)ₙ-NH₂ mit n = 1-20; NH₂-(CH₂)ₙ-CO₂H; oder NH₂-CH₂-(CH₂-O-CH₂)ₙ-CO₂H mit n = 1 bis 10.

7. Verbindung nach Anspruch 1 oder 2, worin Signal ein mit einer organischen Schicht beschichtetes superparamagnetisches Nanopartikel darstellt, das vorteilhafterweise einen Eisenoxid-Kern enthält.

8. Verbindung nach Anspruch 7, worin die organische Schicht eine gem-Bisphosphonat-Schicht ist.

9. Verbindung nach Anspruch 1 oder 2, worin Signal einen Marker für die optische Bildgebung darstellt.

10. Verbindung nach Anspruch 1 oder 2, wobei Signal ein Lipid-Nanopartikel darstellt, das mindestens ein Chelat umfasst, wobei es sich vorteilhafterweise um das Chelat nach Anspruch 5 handelt.

11. Zusammensetzung für die medizinische Bildgebung, die mindestens eine Verbindung der allgemeinen Formel (I) nach einem der Ansprüche 1 bis 10 und einen pharmazeutisch annehmbaren Exzipienten umfasst.

12. Verbindung (I) nach einem der Ansprüche 1 bis 10 für die Verwendung bei einem Verfahren zum Diagnostizieren einer Erkrankung, die mit MUC5AC in Zusammenhang steht, insbesondere ausgewählt aus hereditären Kolorektalkrankheiten, chronisch entzündlichen Erkrankungen des Verdauungstrakts, Populationen, bei denen die Gefahr eines Kolorektalkrebses droht, Überwachungen von Rückfällen bei Patienten, die bereits wegen Kolorektalkrebs behandelt wurden.

13. Verwendung einer Verbindung (I) nach einem der Ansprüche 1 bis 10 zur Herstellung einer Zusammensetzung zum Diagnostizieren einer Erkrankung, die mit MUC5AC in Zusammenhang steht, insbesondere ausgewählt aus hereditären Kolorektalkrankheiten, chronisch entzündlichen Erkrankungen des Verdauungstrakts, Populationen, bei denen die Gefahr eines Kolorektalkrebses droht, Überwachungen von Rückfällen bei Patienten, die bereits wegen Kolorektalkrebs behandelt wurden.

## Claims

1. Compound of general formula (I) below:
Signal-Linker-Peptide (I)
in which:
- Signal represents a signal entity;
- Linker, which may or may not be present, represents a chemical linking component, and
- Peptide represents a peptide comprising an MUC5AC-targeting peptide, the MUC5AC-targeting peptide being chosen from the peptides having the following formula:
a) X1-X2-X3-X4-X5-X6-X7-X8-X9 (1) (SEQ ID No. 1) with:
- X1 absent or chosen from cysteine or methionine,
- X2 chosen from hydroxyproline or proline,
- X3 chosen from threonine or serine,
- X4 chosen from isoleucine, leucine or valine,
- X5 chosen from tyrosine, phenylalanine or tryptophan,
- X6 chosen from hydroxyproline or proline,
- X7 chosen from isoleucine, leucine or valine
- X8 chosen from isoleucine, leucine or valine
- X9 absent or chosen from cysteine or methionine;
b) X10-X11-X12-X13-X14-X15-X16-X17-X18 (2) (SEQ ID No. 4) with:
- X10 absent or chosen from cysteine or methionine,
- X11 chosen from isoleucine, leucine, valine or alanine,
- X12 chosen from threonine or serine,
- X13 chosen from isoleucine, leucine or valine,
- X14 chosen from hydroxyproline or proline,
- X15 chosen from hydroxyproline or proline,
- X16 chosen from isoleucine, leucine or valine,
- X17 chosen from isoleucine, leucine or valine,
- X18 absent or chosen from cysteine or methionine;
c) CALIPPLLC (SEQ ID No. 5);
the pharmaceutically acceptable salts of these compounds of a) or of b).

2. Compound according to Claim 1, wherein Peptide is a peptide of formula CPSIYPLLC (SEQ ID No. 2), peptide PSIYPLL (SEQ ID No. 3) or peptide CALIPPLLC (SEQ ID No. 5).

3. Compound according to Claim 1 or 2, wherein Signal is a chelate optionally coupled to a paramagnetic metal M, M being advantageously chosen from Gd, Mn, Fe, Dy and Tm.

4. Compound according to Claim 1 or 2, wherein Signal is a chelate optionally coupled to a radionuclide ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr or ¹⁶⁶Ho.

5. Compound according to Claim 3 or 4, wherein the chelate is chosen from: DOTA, DTPA, DO3A, HPDO3A, BOPTA, NOTA, PCTA, DOTMA, AAZTA and HOPO, and derivatives thereof.

6. Compound according to any one of Claims 1 to 5, wherein Linker represents:
a) a group of formula: Q1-I-Q2,
in which Q1 and Q2, which may be identical or different, represent O, S, NH, CO₂, -NHCO, CONH, NHCONH, NHCSNH, SO₂NH- or NHSO₂-,
and I represents an alkyl group, alkoxyalkyl group, polyalkoxyalkylene group, alkenyl group, alkynyl group, alkyl group interrupted with one or more squarates, with one or more aryls, advantageously phenyl, or with one or more groups chosen from -NH-, -O-, -CO-, -NH(CO)-, -(CO)NH-, -O(CO)- or -(OC)O-;
b) a group (CH₂)ₙ, (CH₂)ₙ-CO-, -(CH₂)ₙNH-CO- with n = 2 to 10, (CH₂CH₂O)_{q}(CH₂)ᵣ-CO-, (CH₂CH₂O)_{q}(CH₂)ᵣ-NH-CO- with q = 1-10 and r = 2-10, (CH₂)ₙ-CONH-, (CH₂)ₙ-CONH-PEG, (CH₂)ₙ-NH-, squarate, with n = 1 to 5 and advantageously n = 4 or 5, HOOC-CH₂-O-(CH₂)₂-O-(CH₂)₂-O-CH₂-COOH; HOOC-(CH₂)₂-CO₂-(CH₂)₂-OCO-(CH₂)₂-COOH; HOOC-CH(OH)-CH(OH)-COOH; HOOC-(CH₂)ₙ-COOH; NH₂-(CH₂)ₙ-NH₂, with n = 1-20; NH₂-(CH₂)ₙ-CO₂H; or NH₂-CH₂-(CH₂-O-CH₂)ₙ-CO₂H with n = 1 to 10.

7. Compound according to Claim 1 or 2, wherein Signal represents a superparamagnetic nanoparticle coated with an organic layer, advantageously comprising an iron oxide core.

8. Compound according to Claim 7, wherein the organic layer is a gem-bisphosphonate layer.

9. The compound according to Claim 1 or 2, wherein Signal represents a marker for optical imaging.

10. Compound according to Claim 1 or 2, wherein Signal represents a lipid nanoparticle comprising at least one chelate, advantageously the chelate is as defined in claim 5.

11. Composition for medical imaging, comprising at least one compound of general formula (I) according to one of Claims 1 to 10 and a pharmaceutically acceptable excipient.

12. Compound (I) according to one of Claims 1 to 10 intended to be used in a method for diagnosis of an MUC5AC-related pathological condition, in particular chosen from hereditary colorectal diseases, chronic inflammatory bowel diseases, populations at risk of contracting colorectal cancer, and follow-ups of occurrences in patients who have already been treated with colorectal cancer.

13. Use of a compound (I) according to one of Claims 1 to 10 for the preparation of a composition for diagnosis of an MUC5AC-related pathological condition, in particular chosen from hereditary colorectal diseases, chronic inflammatory bowel diseases, populations at risk of contracting colorectal cancer, and follow-ups of occurrences in patients who have already been treated with colorectal cancer.


### SEQUENCE LISTING

<110> GUERBET PROCEDURE
<120> COMPOSES POUR LE DIAGNOSTIC DE MALADIES LIEES A L'EXPRESSION DE MUC5AC
<130> BFF10P0838
<160> 5
<170> PatentIn version 3.4
<210> 1
   <211> 9
   <212> PRT
   <213> Artificial
<220>
   <223> peptide
<220>
   <221> MISC_FEATURE
   <222> (1)..(1)
   <223> Xaa is either absent or represents Cys or Met
<220>
   <221> MISC_FEATURE
   <222> (2)..(2)
   <223> Xaa = Hydroxyproline or Pro
<220>
   <221> MISC_FEATURE
   <222> (3)..(3)
   <223> Xaa = Thr or Ser
<220>
   <221> MISC_FEATURE
   <222> (4)..(4)
   <223> Xaa = Ile or Leu or Val
<220>
   <221> MISC_FEATURE
   <222> (5)..(5)
   <223> Xaa = Tyr or Phe or Trp
<220>
   <221> MISC_FEATURE
   <222> (6)..(6)
   <223> Xaa = Hydroxyproline or Pro
<220>
   <221> MISC_FEATURE
   <222> (7)..(7)
   <223> Xaa = Ile or Leu or Val
<220>
   <221> MISC_FEATURE
   <222> (8)..(8)
   <223> Xaa = Ile or Leu or Val
<220>
   <221> MISC_FEATURE
   <222> (9) .. (9)
   <223> Xaa is either absent or represents Cys or Met
<400> 1
   Xaa Xaa Xaa Xaa Xaa Xaa Xaa Xaa Xaa
   1               5
<210> 2
   <211> 9
   <212> PRT
   <213> Artificial
<220>
   <223> peptide
<400> 2
   Cys Pro Ser Ile Tyr Pro Leu Leu Cys
   1               5
<210> 3
   <211> 7
   <212> PRT
   <213> Artificial
<220>
   <223> peptide
<400> 3
   Pro Ser Ile Tyr Pro Leu Leu
   1               5
<210> 4
   <211> 9
   <212> PRT
   <213> Artificial
<220>
   <223> peptide
<220>
   <221> MISC_FEATURE
   <222> (1)..(1)
   <223> Xaa is either absent or represents Cys or Met
<220>
   <221> MISC_FEATURE
   <222> (2)..(2)
   <223> Xaa = Ile or Leu or Val or Ala
<220>
   <221> MISC_FEATURE
   <222> (3)..(3)
   <223> Xaa = Thr or Ser
<220>
   <221> MISC_FEATURE
   <222> (4)..(4)
   <223> Xaa = Ile or Leu or Val
<220>
   <221> MISC_FEATURE
   <222> (5)..(5)
   <223> Xaa = Hydroxyproline or Pro
<220>
   <221> MISC_FEATURE
   <222> (6)..(6)
   <223> Xaa = Hydroxyproline or Pro
<220>
   <221> MISC_FEATURE
   <222> (7)..(7)
   <223> Xaa = Ile or Leu or Val
<220>
   <221> MISC_FEATURE
   <222> (8)..(8)
   <223> Xaa = Ile or Leu or Val
<220>
   <221> MISC_FEATURE
   <222> (9) .. (9)
   <223> Xaa is either absent or represents Cys or Met
<400> 4
   Xaa Xaa Xaa Xaa Xaa Xaa Xaa Xaa Xaa
   1               5
<210> 5
   <211> 9
   <212> PRT
   <213> Artificial
<220>
   <223> peptide
<400> 5
   Cys Ala Leu Ile Pro Pro Leu Leu Cys
   1               5